(19) **Europäisches Patentamt** / **European Patent Office** / **Office européen des brevets**

(11) **EP 3 742 397 A1**

(12) **EUROPEAN PATENT APPLICATION**

(43) Date of publication:
25.11.2020 Bulletin 2020/48

(51) Int Cl.:
*G06T 7/60* (2017.01)    *G06T 7/62* (2017.01)

(21) Application number: 19176080.0

(22) Date of filing: 23.05.2019

(84) Designated Contracting States:
AL AT BE BG CH CY CZ DE DK EE ES FI FR GB
GR HR HU IE IS IT LI LT LU LV MC MK MT NL NO
PL PT RO RS SE SI SK SM TR
Designated Extension States:
BA ME
Designated Validation States:
KH MA MD TN

(71) Applicant: Koninklijke Philips N.V.
5656 AG Eindhoven (NL)

(72) Inventors:
• ZNAMENSKIY, Dmitry Nikolayevich
5656 AE Eindhoven (NL)

• HEINRICH, Adrienne
5656 AE Eindhoven (NL)
• GALLUCCI, Alessio
5656 AE Eindhoven (NL)
• CIUHU, Calina
5656 AE Eindhoven (NL)
• ZEITOUNY, Mounir
5656 AE Eindhoven (NL)
• KOOIJMAN, Gerben
5656 AE Eindhoven (NL)

(74) Representative: de Haan, Poul Erik et al
Philips International B.V.
Philips Intellectual Property & Standards
High Tech Campus 5
5656 AE Eindhoven (NL)

(54) **ESTIMATING A SURFACE AREA AND/OR VOLUME OF A BODY OR A BODY PART OF A SUBJECT**

(57)     According to an aspect, there is provided a computer-implemented method for estimating a surface area and/or a volume of a body or a body part of a subject. The method comprises obtaining at least one image, wherein the at least one image includes a face of the subject; processing the at least one image to determine values for one or more facial image parameters for the face of the subject; determining values for one or more characteristics of the subject, wherein the one or more characteristics comprises one or more of age of the subject, weight of the subject, height of the subject and gender of the subject; using a facial parametric model and the determined values for the one or more facial image parameters to determine values for one or more facial shape parameters for the face of the subject, wherein the facial parametric model relates specific values for one or more facial image parameters to a respective 3D representation of a face having respective values for the one or more facial shape parameters; using a prediction model with the determined values for the one or more characteristics and the determined values of the one or more facial shape parameters to predict a 3D representation of the full body of the subject; and analyzing the predicted 3D representation of the full body of the subject to estimate the surface area and/or the volume of the body or body part of the subject. A corresponding apparatus and computer program product is also provided.

Fig. 2

**Description**

FIELD OF THE INVENTION

This disclosure relates to estimating a surface area and/or volume of a body or a body part of a subject, for example for use in a treatment operation that is to be performed on the body or the body part, and in particular to a computer-implemented method, apparatus and computer program product for estimating a surface area and/or volume of a body or a body part of a subject.

BACKGROUND OF THE INVENTION

In adult humans the body or skin surface area (BSA) of a subject can be anywhere in a wide range, typically from about 1.4 to 2.6 square meters ($m^2$). Body volume (BV) of a subject can likewise vary over a wide range.

The use of the three dimensional (3D) human body shape measurements or models has the potential to change the way that subjects interact with the world in a wide variety of ways. Applications of this technology could be helpful in several fields such as healthcare, online shopping and the textile industry. For example, in the healthcare domain, the knowledge of the 3D body shape can help in the assessment of the Psoriasis Area and Severity Index (PASI), or dosing chemotherapy or other drugs according to the BSA.

Clearly for these applications it is desirable for the estimation of the BSA, BV and body shape prediction to be as accurate as possible. One of the most accurate techniques for estimating BSA or BV or estimating the surface area and/or volume of a particular body part uses full body 3D scanning of the subject and processing to identify different body parts. However, the technology required for full body 3D scanning is expensive and typically not available in, for example, domestic environments, and therefore impacts the scenarios in which BSA, BV or body part surface area and/or volume can potentially be used.

It is therefore desirable to be able to estimate BSA, BV or the surface area and/or volume of particular body parts in a more cost effective and simple way, for example using information that is readily available to a subject and/or obtainable by the subject.

Some techniques exist in which a 3D model of the body of a subject or of a particular body part of a subject can be determined from available metadata, such as age, gender, volume, blood pressure, number of children, body mass index (BMI), etc., and body measurements, such as height, leg length, arm circumference, etc., although often these techniques do not provide surface area and/or volume values that are sufficiently accurate for many applications. One example of such a technique is found in "Estimating 3D human shapes from measurements" by S. Wuhrer and C. Shu, Mach. Vis. Appl., vol. 24, no. 6, pp. 1133-1147, 2013.

Therefore there is a need for improvements in estimating a surface area and/or volume of a body part or body of a subject without requiring a 3D body scan or 3D body part scan of the subject.

SUMMARY OF THE INVENTION

According to a first specific aspect, there is provided a computer-implemented method for estimating a surface area and/or a volume of a body or a body part of a subject. The method comprises obtaining at least one image, wherein the at least one image includes a face of the subject; processing the at least one image to determine values for one or more facial image parameters for the face of the subject; determining values for one or more characteristics of the subject, wherein the one or more characteristics comprises one or more of age of the subject, weight of the subject, height of the subject and gender of the subject; using a facial parametric model and the determined values for the one or more facial image parameters to determine values for one or more facial shape parameters for the face of the subject, wherein the facial parametric model relates specific values for one or more facial image parameters to a respective 3D representation of a face having respective values for the one or more facial shape parameters; using a prediction model with the determined values for the one or more characteristics and the determined values of the one or more facial shape parameters to predict a 3D representation of the full body of the subject; and analyzing the predicted 3D representation of the full body of the subject to estimate the surface area and/or the volume of the body or body part of the subject. Thus, the surface area and/or volume of the body or body part(s) can be determined just using one or more images of the subject and one or more of the age, weight, height and gender.

In some embodiments, the facial parametric model is a linear model. In some embodiments, the prediction model is a non-linear model, e.g. a non-linear regression model, such as a cubic polynomial.

In some embodiments, the step of using the prediction model to predict the 3D representation of the full body comprises using the prediction model to predict values of one or more body shape parameters from the determined values for the one or more characteristics and the determined values of the one or more facial shape parameters; and using a body parametric model and the predicted values of the one or more body shape parameters to predict the 3D

**EP 3 742 397 A1**

representation of the full body, wherein the body parametric model relates specific values for the one or more body shape parameters to a respective 3D representation of a body. In these embodiments, the body parametric model can be a linear model.

In some embodiments, the method further comprises determining the prediction model from a population dataset, wherein the population dataset comprises 3D scans of a plurality of test subjects and values for the one or more characteristics for each of the test subjects, and the prediction model is determined by: registering a body parametric model to each of the 3D scans, wherein the body parametric model relates a 3D representation of a body to specific values of one or more body shape parameters; determining values of the one or more body shape parameters for each of the registered body parametric models; registering a facial parametric model to each of the 3D scans, wherein the facial parametric model relates a 3D representation of a face to specific values of one or more facial shape parameters; determining values of the one or more facial shape parameters for each of the registered facial parametric models; and forming the prediction model from the determined values of the one or more body shape parameters, determined values of the one or more facial shape parameters and values for the one or more characteristics for each of the test subjects.

In some embodiments, the prediction model is specific to the body part of the subject for which the surface area and/or the volume is to be estimated, wherein the prediction model predicts the 3D representation of the full body based on determined values of the one or more facial shape parameters and a respective subset of the one or more characteristics. In this way the prediction model can be customized to the body part(s) of interest, requiring values for only some of the characteristics.

In some embodiments, the step of forming the prediction model comprises: forming a plurality of candidate prediction models, wherein each candidate prediction model uses a respective subset of the one or more characteristics; evaluating an accuracy of each of the candidate prediction models in predicting the 3D representation of the full body or a body part or body parts; and forming the prediction model as a candidate prediction model that provides one of: a highest accuracy of the candidate prediction models, and/or a sufficient accuracy with a minimum number of characteristics.

In some embodiments, the method further comprises: receiving an indication of the body or the body part of the subject for which the surface area and/or the volume is to be estimated; wherein the step of analyzing comprises analyzing the predicted 3D representation of the full body of the subject to estimate the surface area and/or the volume of the indicated body or body part of the subject.

In these embodiments, the method can further comprise: requesting an input indicating the body or body part of the subject for which the surface area and/or the volume is to be estimated.

In these embodiments, the indication can be received as an input corresponding to a body part or body parts displayed on a user interface.

In some embodiments, the method can further comprise: requesting an input indicating the values of the respective subset of the one or more characteristics used by the prediction model for the indicated body part.

In some embodiments, the step of forming the prediction model can be performed after receiving the indication. In this way, the user is able to indicate any desired body part(s) or combination of body part(s) for which the surface area and/or volume is to be estimated, and a prediction model that is suitable for modelling that/those body part(s) can be determined.

In some embodiments, the body part of the subject for which the surface area and/or the volume is to be estimated comprises a plurality of non-contiguous body parts.

In some embodiments, the step of determining values for the plurality of characteristics comprises processing the at least one image to determine the values for one or more of the plurality of characteristics. This has the advantage that it is not necessary for the values of the one or more characteristics to be manually input by a user or the subject.

In alternative embodiments, the step of determining values for the one or more characteristics comprises receiving an input from the subject indicating the values for one or more of the one or more characteristics.

In some embodiments, the at least one characteristic of the subject is a plurality of characteristics of the subject comprising two or more of age, weight, height and gender.

In some embodiments, the body part is one or more of an arm, both arms, a leg, both legs, a foot, both feet, a hand, both hands, a palm of a hand, both palms, a breast or both breasts, waist, hips, chest, torso, abdomen, and back.

According to a second aspect, there is provided a computer-implemented method of providing feedback on a treatment operation by a treatment device. The method comprises: estimating the surface area and/or volume of a body or a body part of a subject that is to be treated in a treatment operation using the treatment device according to the first aspect or any embodiment thereof; and using the estimated surface area and/or volume to determine feedback on the treatment operation.

In some embodiments, the feedback is any one of: feedback on progress of the treatment operation; a number of treatments required to treat the body or body part in the treatment operation; a number of treatments remaining required to treat the body or body part in the treatment operation; guidance to a user of the treatment device to treat the body or body part.

According to a third aspect, there is provided a computer program product comprising a computer readable medium having computer readable code embodied therein, the computer readable code being configured such that, on execution by a suitable computer or processor, the computer or processor is caused to perform the method according to the first aspect, the second aspect, or any embodiments thereof.

According to a fourth aspect, there is provided an apparatus for estimating a surface area and/or a volume of a body or a body part of a subject. The apparatus comprises a processing unit configured to obtain at least one image from an imaging unit, wherein the at least one image includes a face of the subject; process the at least one image to determine values for one or more facial image parameters for the face of the subject; determine values for one or more characteristics of the subject, wherein the one or more characteristics comprises one or more of age of the subject, weight of the subject, height of the subject and gender of the subject; use a facial parametric model and the determined values for the one or more facial image parameters to determine values for one or more facial shape parameters for the face of the subject, wherein the facial parametric model relates specific values for one or more facial image parameters to a respective 3D representation of a face having respective values for the one or more facial shape parameters; use a prediction model with the determined values for the one or more characteristics and the determined values of the one or more facial shape parameters to predict a 3D representation of the full body of the subject; and analyze the predicted 3D representation of the full body of the subject to estimate the surface area and/or the volume of the body or body part of the subject. Thus, the surface area and/or volume of the body or body part(s) can be determined just using one or more images of the subject and one or more of the age, weight, height and gender.

In some embodiments, the facial parametric model is a linear model. In some embodiments, the prediction model is a non-linear model, e.g. a non-linear regression model, such as a cubic polynomial.

In some embodiments, the processing unit is configured to use the prediction model to predict values of one or more body shape parameters from the determined values for the one or more characteristics and the determined values of the one or more facial shape parameters; and use a body parametric model and the predicted values of the one or more body shape parameters to predict the 3D representation of the full body, wherein the body parametric model relates specific values for the one or more body shape parameters to a respective 3D representation of a body. In these embodiments, the body parametric model can be a linear model.

In some embodiments, the processing unit is further configured to determine the prediction model from a population dataset, wherein the population dataset comprises 3D scans of a plurality of test subjects and values for the one or more characteristics for each of the test subjects, and the processing unit is configured to determine the prediction model by: registering a body parametric model to each of the 3D scans, wherein the body parametric model relates a 3D representation of a body to specific values of one or more body shape parameters; determining values of the one or more body shape parameters for each of the registered body parametric models; registering a facial parametric model to each of the 3D scans, wherein the facial parametric model relates a 3D representation of a face to specific values of one or more facial shape parameters; determining values of the one or more facial shape parameters for each of the registered facial parametric models; and forming the prediction model from the determined values of the one or more body shape parameters, determined values of the one or more facial shape parameters and values for the one or more characteristics for each of the test subjects.

In some embodiments, the prediction model is specific to the body part of the subject for which the surface area and/or the volume is to be estimated, wherein the prediction model predicts the 3D representation of the full body based on determined values of the one or more facial shape parameters and a respective subset of the one or more characteristics. In this way the prediction model can be customized to the body part(s) of interest, requiring values for only some of the characteristics.

In some embodiments, the processing unit is configured to form the prediction model by: forming a plurality of candidate prediction models, wherein each candidate prediction model uses a respective subset of the one or more characteristics; evaluating an accuracy of each of the candidate prediction models in predicting the 3D representation of the full body or a body part or body parts; and forming the prediction model as a candidate prediction model that provides one of: a highest accuracy of the candidate prediction models, and/or a sufficient accuracy with a minimum number of characteristics.

In some embodiments, the processing unit is further configured to: receive an indication of the body or the body part of the subject for which the surface area and/or the volume is to be estimated; wherein the processing unit is configured to analyze the predicted 3D representation of the full body of the subject to estimate the surface area and/or the volume of the indicated body or body part of the subject.

In these embodiments, the processing unit is further configured to: request an input indicating the body or body part of the subject for which the surface area and/or the volume is to be estimated.

In these embodiments, the indication can be received as an input corresponding to a body part or body parts displayed on a user interface.

In some embodiments, the processing unit is further configured to: request an input indicating the values of the respective subset of the one or more characteristics used by the prediction model for the indicated body part.

In some embodiments, the processing unit is configured to form the prediction model after receiving the indication. In this way, the user is able to indicate any desired body part(s) or combination of body part(s) for which the surface area and/or volume is to be estimated, and a prediction model that is suitable for modelling that/those body part(s) can be determined.

In some embodiments, the body part of the subject for which the surface area and/or the volume is to be estimated comprises a plurality of non-contiguous body parts.

In some embodiments, the processing unit is configured to determine values for the plurality of characteristics by processing the at least one image to determine the values for one or more of the plurality of characteristics. This has the advantage that it is not necessary for the values of the one or more characteristics to be manually input by a user or the subject.

In alternative embodiments, the processing unit is configured to determine values for the one or more characteristics by receiving an input from the subject indicating the values for one or more of the one or more characteristics.

In some embodiments, the at least one characteristic of the subject is a plurality of characteristics of the subject comprising two or more of age, weight, height and gender.

In some embodiments, the body part is one or more of an arm, both arms, a leg, both legs, a foot, both feet, a hand, both hands, a palm of a hand, both palms, a breast or both breasts, waist, hips, chest, torso, abdomen, and back.

According to a fifth aspect, there is provided an apparatus for providing feedback on a treatment operation by a treatment device. The apparatus comprises a processing unit configured to: estimate the surface area and/or volume of a body or a body part of a subject that is to be treated in a treatment operation using the treatment device according to the fourth aspect or any embodiment thereof; and use the estimated surface area and/or volume to determine feedback on the treatment operation.

In some embodiments, the feedback is any one of: feedback on progress of the treatment operation; a number of treatments required to treat the body or body part in the treatment operation; a number of treatments remaining required to treat the body or body part in the treatment operation; guidance to a user of the treatment device to treat the body or body part.

According to a sixth aspect, there is provided a system, comprising an apparatus according to the fourth aspect, fifth aspect, or any embodiment thereof; and an imaging unit for obtaining at least one image.

These and other aspects will be apparent from and elucidated with reference to the embodiment(s) described hereinafter.

BRIEF DESCRIPTION OF THE DRAWINGS

Exemplary embodiments will now be described, by way of example only, with reference to the following drawings, in which:

Fig. 1 is a block diagram of an exemplary system comprising an apparatus according to an embodiment and an imaging unit;
Fig. 2 illustrates various body shape parameters that can typically be measured or obtained for a body;
Fig. 3 illustrates five different body parts for which the surface area and/or volume can be estimated using the techniques described herein;
Fig. 4 shows template meshes for a body and a face;
Fig. 5 shows two graphs that illustrate the standard deviation of PCA coefficients for the body and face;
Fig. 6 is a table that illustrates the mean absolute vertices errors for four different models for the full body mask;
Fig. 7 is a table that illustrates the mean absolute vertices errors for four different models for the waist band mask;
Fig. 8 is a table that illustrates the mean absolute vertices errors for four different models for the hips band mask;
Fig. 9 is a table that illustrates the mean absolute vertices errors for four different models for the breasts mask;
Fig. 10 is a table that illustrates the mean absolute vertices errors for four different models for the legs mask;
Fig. 11 is a table that illustrates the mean absolute vertices errors for four different models for the full body mask for different combinations of the features age, gender, weight and height;
Fig. 12 is a table that illustrates the mean absolute vertices errors for four different models for the waist band mask for different combinations of the features age, gender, weight and height;
Fig. 13 is a table that illustrates the mean absolute vertices errors for four different models for the hips band mask for different combinations of the features age, gender, weight and height;
Fig. 14 is a table that illustrates the mean absolute vertices errors for four different models for the breasts mask for different combinations of the features age, gender, weight and height;
Fig. 15 is a table that illustrates the mean absolute vertices errors for four different models for the legs mask for different combinations of the features age, gender, weight and height;
Fig. 16 is a flow chart illustrating a method according to an exemplary embodiment; and

Fig. 17 is a flow chart illustrating a method according to another exemplary embodiment.

DETAILED DESCRIPTION OF EMBODIMENTS

As noted above, there are various applications for which information on the (total) body surface area (BSA), body volume (BV) or surface area and/or volume of a body part (e.g. a leg, both legs, arm, both arms, chest, back, torso, etc.) is useful. However, in many of these applications it is desirable to be able to obtain reliable estimates of body/skin surface area/volume without having to perform full body 3D scanning of the subject or the relevant body part(s), since the technology required for full body 3D scanning is expensive, both in terms of cost and processing resources, and typically not available in domestic environments.

One of the problems in the efficient processing of full body 3D models is the high volume of data. The cost and volume of the data required can be significantly reduced by the system 'learning' a statistical representation of the 'generic' human shape space, which means that only sparse data about the subject combined with the learned space is needed to reconstruct a full body 3D model of the subject instead of a dense representation of the subject themselves. The techniques described herein provide for estimates of the BSA, BV, surface area and/or volume of a body part(s) for a subject to be obtained using a statistical representation of a generic human shape space, and limited, but easily obtainable, additional information about the subject. In particular, it has been found that features of the shape of the face of the subject (which can be observed or inferred from an image of the face of the subject), in combination with values of one or more characteristics of the subject, such as age, weight, height and/or gender, can provide sufficiently reliable estimates of the BSA, BV, surface area and/or volume of a body part(s) for a subject.

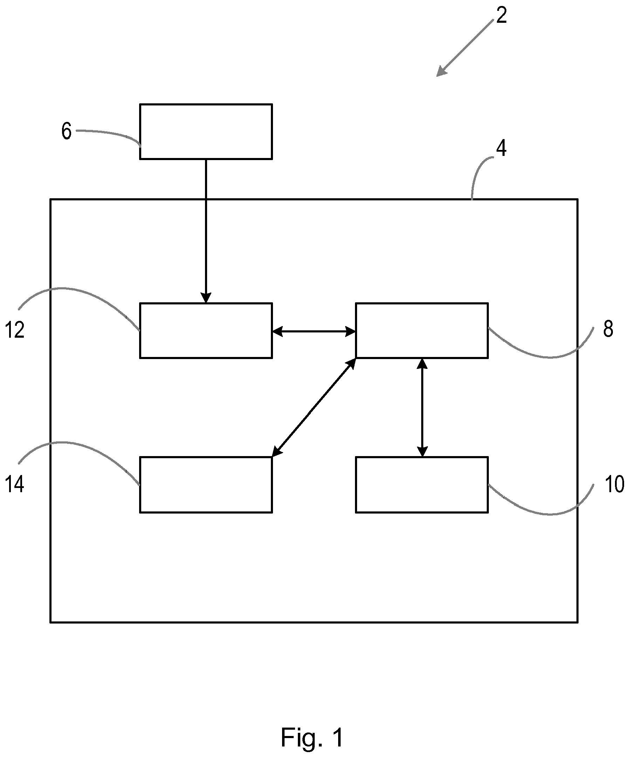

Fig. 1 is a block diagram of an exemplary system 2 that can be used to estimate the surface area and/or volume of the body or a body part of a subject. The system 2 comprises an apparatus 4 for implementing exemplary embodiments of the techniques described herein and the system 2 can also comprise an imaging unit 6 for obtaining an image of a subject's face. In this illustrated embodiment, the apparatus 4 is shown as being physically separate from the imaging unit 6, but it will be appreciated that in some embodiments of the system 2 the apparatus 4, or the functionality provided by the apparatus 4, can be part of, or integral with, the imaging unit 6.

The imaging unit 6 may include or be any suitable component for capturing an image, for example a charge-coupled device (CCD) and may include one or more lenses and/or mirrors. In some embodiments, the imaging unit 6 is the camera, or one of the cameras, in an electronic device such as a smartphone, a smartwatch, a tablet computer, a laptop, a digital camera or a smart mirror. The imaging unit 6 is able to output an obtained image, or series of obtained images to the apparatus 4.

The apparatus 4 comprises a processing unit 8 that is for estimating the surface area and/or volume of a body or particular body part according to the techniques described herein. The processing unit 8 can be implemented in numerous ways, with software and/or hardware, to perform the various functions described herein. The processing unit 8 may comprise one or more microprocessors or digital signal processor (DSPs) that may be programmed using software or computer program code to perform the required functions and/or to control components of the processing unit 8 to effect the required functions. The processing unit 8 may be implemented as a combination of dedicated hardware to perform some functions (e.g. amplifiers, pre-amplifiers, analog-to-digital convertors (ADCs) and/or digital-to-analog convertors (DACs)) and a processor (e.g., one or more programmed microprocessors, controllers, microcontrollers, DSPs and associated circuitry) to perform other functions. Examples of components that may be employed in various embodiments of the present disclosure include, but are not limited to, conventional microprocessors, DSPs, application specific integrated circuits (ASICs), and field-programmable gate arrays (FPGAs).

The processing unit 8 is connected to a memory unit 10 (in alternative embodiments the memory unit 10 can be part of the processing unit 8), and the memory unit 10 can store data, information and/or signals for use by the processing unit 8 in estimating the surface area and/or volume of a body or particular body part. For example the memory unit 10 can store one or more images of a subject. In some implementations the memory unit 10 stores computer-readable code that can be executed by the processing unit 8 so that the processing unit 8 performs one or more functions, including the operations described herein. The memory unit 10 can comprise any type of non-transitory machine-readable medium, such as cache or system memory including volatile and non-volatile computer memory such as random access memory (RAM) static RAM (SRAM), dynamic RAM (DRAM), read-only memory (ROM), programmable ROM (PROM), erasable PROM (EPROM) and electrically erasable PROM (EEPROM), implemented in the form of a memory chip, an optical disk (such as a compact disc (CD), a digital versatile disc (DVD) or a Blu-Ray disc), a hard disk, a tape storage solution, or a solid state device, including a memory stick, a solid state drive (SSD), a memory card, etc.

The apparatus 4 can also include interface circuitry 12. The interface circuitry 12 can enable the apparatus 4 to communicate with the imaging unit 6 (e.g. to receive images obtained by the imaging unit 6). In embodiments where the apparatus 4, or the functionality provided by the apparatus 4, is part of or integral with the imaging unit 6, the interface circuitry 12 in the apparatus 4 may be omitted.

The interface circuitry 12 in the apparatus 4 is for enabling a data connection to and/or data/information exchange with another device/apparatus/unit, including the imaging unit 6, if present in the system 2. The connection may be direct or indirect (e.g. via the Internet), and thus the interface circuitry 12 can enable a connection between the apparatus 4 and a network, such as the Internet, or directly between the apparatus 4 and another device/apparatus/unit, via any desirable wired or wireless communication protocol. For example, the interface circuitry 12 can operate using WiFi, Bluetooth, Zigbee, or any cellular communication protocol (including but not limited to Global System for Mobile Communications (GSM), Universal Mobile Telecommunications System (UMTS), Long Term Evolution (LTE), LTE-Advanced, etc.). In the case of a wireless connection, the interface circuitry 12 (and thus the apparatus 4) may include one or more suitable antennas for transmitting/receiving over a transmission medium (e.g. the air). Alternatively, in the case of a wireless connection, the interface circuitry 12 may include means (e.g. a connector or plug) to enable the interface circuitry 12 to be connected to one or more suitable antennas external to the apparatus 4 for transmitting/receiving over a transmission medium (e.g. the air). The interface circuitry 12 is connected to the processing unit 8 to enable information or data received by the interface circuitry 12 to be provided to the processing unit 8, and/or to enable information or data from the processing unit 8 (e.g. an estimate of the surface area of the body or a body part) to be transmitted by the interface circuitry 12.

In some embodiments, the apparatus 4 comprises a user interface 14 that includes one or more components that enables a user of apparatus 4 (e.g. the subject) to input information, data and/or commands into the apparatus 4, such as an indication of the part of the body that the surface area and/or volume should be estimated for, and/or enables the apparatus 4 to output information or data to the user of the apparatus 4, for example the estimate of the surface area and/or volume of the body or body part. The user interface 14 can comprise any suitable input component(s), including but not limited to a keyboard, keypad, one or more buttons, switches or dials, a mouse, a track pad, a touchscreen, a stylus, a camera, a microphone, etc., and the user interface 14 can comprise any suitable output component(s), including but not limited to a display screen, one or more lights or light elements, one or more loudspeakers, a vibrating element, etc.

The apparatus 4 can be any type of electronic device or computing device. In some implementations, the apparatus 4 can be, or be part of, a laptop, a tablet, a computer, a smartphone, a smartwatch, a smart mirror, etc., or other type of electronic device that can be present or used in a home or care environment of the subject/user. In other implementations, the apparatus 4 is an apparatus that is remote from the subject/user, and remote from the home or care environment of the subject/user. For example, the apparatus 4 can be a server, for example a server in a data center (also referred to as being 'in the cloud').

It should be noted that Fig. 1 only shows the elements of system 2 and apparatus 4 that are useful for implementing the techniques described herein, and a typical system 2 and apparatus 4 will include further elements. For example the apparatus 4 will also include a power supply, such as a battery, or elements enabling the apparatus 4 to be connected to a mains power supply.

As noted above, the techniques described herein provide for estimates of the BSA, BV, surface area and/or volume of a body part(s) for a subject to be obtained using a statistical representation of a generic human shape space, in particular a body parametric model, along with features of the shape of the face of the subject (which can be observed or inferred from an image of the face of the subject) and values of one or more characteristics of the subject, such as age, weight, height and/or gender. In particular, one or more images of the face of the subject are processed to determine values for one or more facial image parameters, and a facial parametric model is used to determine one or more facial shape parameters for the subject from the facial image parameters determined from the image(s). The facial shape parameter(s) are input into a prediction model along with the values of the one or more characteristics to determine a 3D representation of the full body. The prediction model predicts values for one or more body shape parameters from the specific values for the one or more characteristics and specific values for the one or more facial shape parameters. A body parametric model (which can be part of the prediction model), is used to determine a 3D representation of the full body of the subject based on the determined values for the one or more body shape parameters. The surface area and/or volume of the full body and/or one or more body parts can be determined from the 3D representation of the full body.

In some preferred embodiments, the techniques described herein enable a body part or body parts of interest to be selected (e.g. by a user using a graphical user interface), and the surface area and/or volume of that/those selected body part(s) estimated. In some further embodiments, as the body part or body parts of interest can be selected dynamically by a user, following the selection of body part(s), a prediction model can be determined that is specific to estimating the surface area and/or volume and that is to receive specific one(s) of the characteristics as inputs.

The following description provides some further details of a facial parametric model, body parametric model and prediction model that can be used in embodiments of the techniques described herein, and in particular indicates how an exemplary facial parametric model and an exemplary body parametric model can be derived from a population dataset that includes data (3D scans) for a population of test subjects, and the effectiveness of these exemplary models in estimating surface area of a full body or a body part based on values of various input parameters. Those skilled in the art will appreciate that the techniques and information provided below can be readily adapted to estimate volume of a full body or body part based on values of various input parameters. In particular, the surface area is estimated from a 3D representation of the full body of the subject of interest, and it is also possible to estimate volume from such a 3D representation.

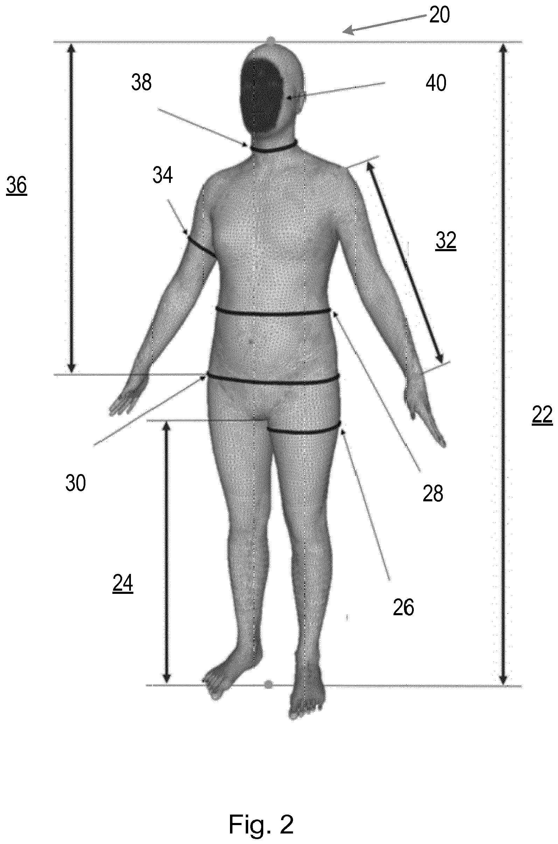

Firstly, Fig. 2 illustrates various body shape parameters that can typically be measured or obtained for a body 20. Thus, typical body shape parameters include height 22, leg length 24, leg (quadriceps) circumference 26, waist circumference 28, hips circumference 30, arm length 32, arm circumference 34, upper body height 36, neck circumference 38 and facial shape parameters 40. Other body shape parameters can be measured or obtained if required.

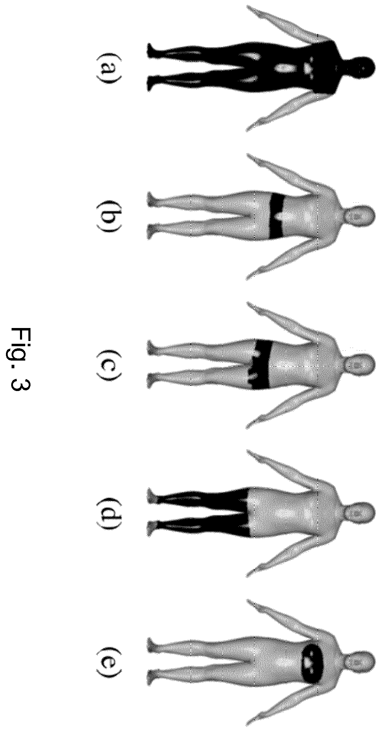

Fig. 3 illustrates five different body parts for which the surface area and/or volume can be estimated using the techniques described herein (although the techniques are not limited just to these body parts, and for example the surface area and/or volume can be estimated for other body parts, portions of these five body parts (e.g. lower leg rather than the full leg) or any combination of different parts of the body). The body parts shown in Fig. 3 are also referred to as segmentation masks (the black portions of the figures), and thus Fig. 3(a) shows a full body mask, Fig. 3(b) shows a waistband mask, Fig. 3(c) shows a hips band mask, Fig. 3(d) shows a legs mask and Fig. 3(e) shows a breasts mask. It will be noted that the full body mask shown in Fig. 3(a) excludes the arms. As noted below, this is due to some implementations of the body parametric model being derived from a population dataset where the test subjects in the population did not use the same pose with their arms when their body shape information was obtained. Those skilled in the art will appreciate from the following that a full body mask that includes the arms can be derived where the population dataset includes test subjects in an appropriate pose. Alternatively, it is possible to analyze a population dataset using a skeleton model, a skinning model or a pose model in order to account for the test subjects in the population dataset being in different poses. A skinning model is described in "SMPL: A skinned multi-person linear model" by Loper, M., Mahmood, N., Romero, J., Pons-Moll, G., & Black, M. J., ACM transactions on graphics (TOG), 34(6), 248. The models can then be used to convert the test subjects into the desired (or a consistent) pose, and the body parametric model can be derived from the converted population dataset.

The facial parametric model and body parametric model can be developed according to the methods described in "The space of human body shapes: reconstruction and parameterization from range scans" (by B. Allen, B. Curless, and Z. Popovic, ACM Transactions on Graphics, 2003) and "A morphable model for the synthesis of 3D faces" (by V. Blanz and T. Vetter, Proceedings of the 26th annual conference on Computer graphics and interactive techniques, 1999).

A facial parametric model can be derived using 3D scans of a large number (e.g. 3000) of different faces (i.e. faces of different shapes, sizes, ages, genders, weights, expressions, etc.). A body parametric model can be derived using 3D scans of a large number (e.g. 4000) of full bodies (including the face). In the present example, the 3D scans were of test subjects that were standing in a 'tree' position (i.e. standing upright with arms out from the body). Databases containing suitable population datasets (i.e. 3D scans of faces and full bodies) are available to those skilled in the art, for example the 'Size China Dataset' described in "Measuring Chinese heads and faces", by R. Ball and J. Molenbroek, Proceedings of the 9th International Congress of Physiological Anthropology, Human diversity: design for life, 2008, and the CAESAR dataset described in "The CAESAR project: a 3-D surface anthropometry survey" by K. Robinette, H. Daanen, and E. Paquet, Second International Conference on 3-D Digital Imaging and Modeling (Cat. No.PR00062), 1999.

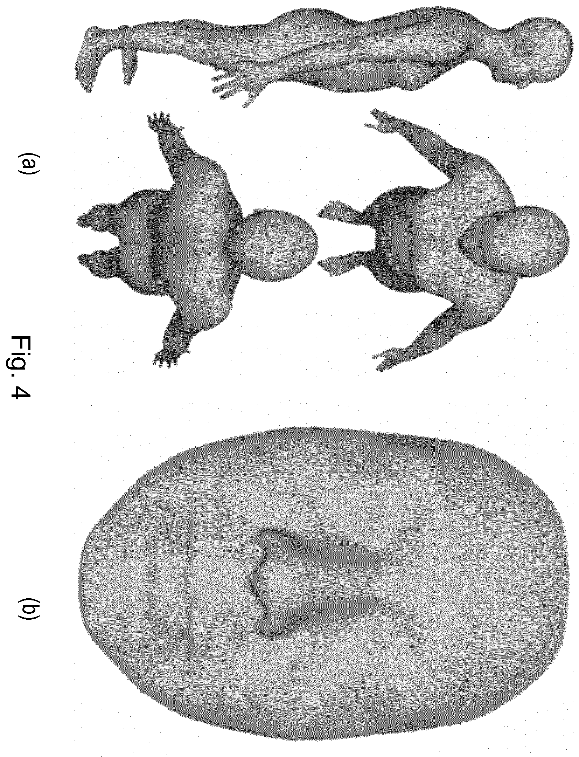

Briefly, respective template meshes for the body and face are registered into the 3D scans of the bodies and faces respectively to form registered models, and the registered models are encoded into the selected body parametric model parameters and facial parametric model parameters. A mesh represents, in a discrete form, the surface of the template or the 3D scans. Fig. 4 shows a template mesh for a body (Fig. 4(a)) and a template mesh for a face (Fig. 4(b)).

Registration - In order to register every face and full body template mesh, non-rigid registration techniques can be used. Such techniques are known from, for example, "Registration of 3d point clouds and meshes: A survey from rigid to Nonrigid" (by G. K. Tam, Z. Q. Cheng, Y. K. Lai, F. C. Langbein, Y. Liu, D. Marshall, R. R. Martin, X. F. Sun, and P. L. Rosin, IEEE Transactions on Visualization and Computer Graphics, 2013), "A survey on shape correspondence" (by O. van Kaick, H. Zhang, G. Hamarneh, and D. Cohen-Or, Eurographics Symposium on Geometry Processing, 2011) and "On Computing Mapping of 3D Objects" (by X. Li and S. S. Iyengar, ACM Computing Surveys, 2014).

A template mesh with about $N_P \approx 53000$ vertices can be used for the body, as shown in Fig. 4(a), although those skilled in the art will appreciate that the number of vertices can be substantially different. A template mesh with about $N_Q \approx 23000$ vertices can be used for the face, as shown in Fig. 4(b), although again, those skilled in the art will appreciate that the number of vertices can be different.

Both template meshes can then be used to register the full population dataset that includes the 3D full body scans. The quality of the registration can be assessed via visual inspection and other measures (e.g. as described in the paper "Registration of 3d point clouds and meshes: A survey from rigid to Nonrigid"). For around $N \approx 3750$ full body 3D scans, both registrations show low fit error (e.g. below 0.5 millimeters (movement measurements) Root Mean Squared Error (RMSE) as surfaces distance for the registration of the facial mesh, and below 1.0mm RMSE for the registration of the full body).

Registration leads to a representation of each test subject in the population dataset as two morphed template meshes. Let $v_{i,j}^r \in \mathbb{R}^3$ be the full body morphed coordinates of vertex $j \in N_P$ for population subject $i \in N$. Furthermore, the morphed coordinates of all vertices of scan $i \in N$ can be written as a single flattened vector, stacking all vertices' coordinates together, as

$$p_i^r = \left(v_{i,1}^r, v_{i,2}^r, \dots, v_{i,N_P}^r\right) \in \mathbb{R}^{3N_P} \tag{1}$$

where $i$ is the test subject, $r$ denotes registered data rather than the (raw) subject data, $N_P$ represents the total number of vertices for the body template and $p_i^r$ represents all vertices of test subject $i$. Collecting all participants into a rectangular matrix results in

$$P_r = (p_1^r; p_2^r; \dots; p_N^r)' \in \mathbb{R}^{N \times 3N_P} \tag{2}$$

where $P_r$ is a matrix including all test subjects and all vertices for the full body. In the same way, the definition of the face representation is

$$Q_r = (q_1^r; q_2^r; \dots; q_N^r)' \in \mathbb{R}^{N \times 3N_Q} \tag{3}$$

where $Q_r$ is a matrix including all test subjects and all vertices for the face and $N_r$ represents the total number of vertices for the face template.

<u>Parametric spaces</u> - The registered meshes can be parametrized with Principal Component Analysis (PCA) transformation, using a plurality of (e.g. 200) Eigenvectors for the body and a plurality (e.g. 180) Eigenvectors for the face. Those skilled in the art will appreciate that different numbers of Eigenvectors can be used. The PCA transformation can be written in matrix form as

$$P_r = \bar{P}_r + YD' + E_r \tag{4}$$

where $\bar{P}_r \in \mathbb{R}^{N \times 3N_P}$ is the matrix of $N$ times repeated average mesh coordinates

$$\bar{p} = \left(\bar{p}_{1_x}, \bar{p}_{1_y}, \dots, \bar{p}_{N_{P_z}}\right) \in \mathbb{R}^{3N}, \quad \bar{p}_{j_x} = \frac{\sum_i P_r(i, j_x)}{N_P}, \tag{5}$$

$D \in \mathbb{R}^{3N_P x 200}$ is the reduced Eigenvectors matrix, composed of the 200 'principal' Eigenvectors (i.e. Eigenvectors with highest Eigenvalues) of the covariance matrix $(P_r - \bar{P}_r)'(P_r - \bar{P}_r)$, $Y \in \mathbb{R}^{N \times 200}$ is the reduced matrix of PCA coefficients, and $E_r \in \mathbb{R}^{3N_P}$ is the residual error, i.e.

$$P_r \approx P = \bar{P}_r + YD' \tag{6}$$

The transformation in equation (6) gives a compact representation of 53000 3D vectors of vertex coordinates $P_r$ with the 200-dimensional PCA coefficient vectors Y. In the same way, the PCA transformation is applied to the registered facial meshes:

$$Q_r \approx Q = \overline{Q_r} + X_Q D_Q' \tag{7}$$

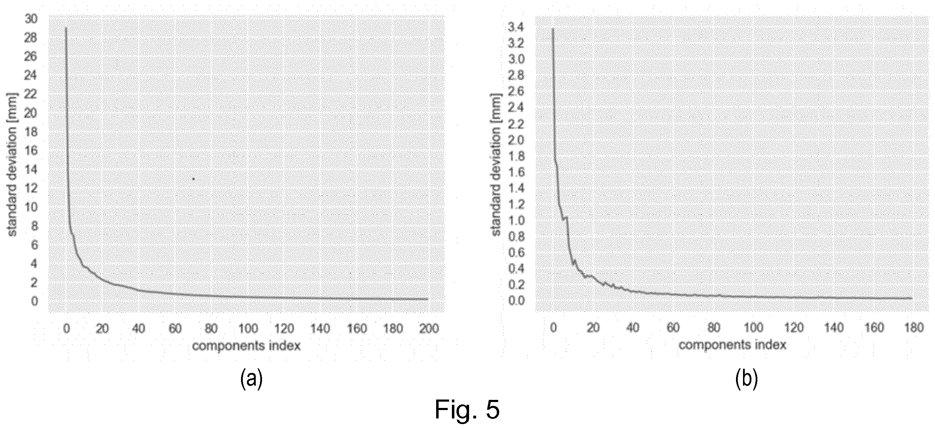

where $\overline{Q}_r \in \mathbb{R}^{Nx3N_Q}$ is the matrix of $N$ times repeated average mesh coordinates $D_Q$ consists of the 180 'principal' eigenvectors of the covariance matrix $(Q_r - \overline{Q}_r)'(Q_r - \overline{Q}_r)$, $X_Q \in \mathbb{R}^{N \times 180}$ are the facial PCA coefficients. The results of the encoding for both models is shown in Fig. 5. Fig. 5 shows the significance of the encoding, i.e. the standard deviation of the PCA coefficients for the body (Fig. 5(a)) and for the face (Fig. 5(b)). Using 200 principal components for the body and 180 for the face is a heuristic decision that seeks a compromise between the requirements to represent all shape spaces adequately and to not encode noise, although those skilled in the art will appreciate that other numbers of principal components can be used. The standard deviation of the last PCA body shape component is 0.18mm and for the face it is 0.025mm. The residual error between $P_r$ and $P$, computed using equation (15) below and explained in the section entitled 'Fitness measures', is less than 2.5mm. Similarly, the residual error for the face is less than 1.0 mm.

<u>Prediction model</u> - This section explains how the body shape coefficients $Y$ are predicted using the subject's features, denoted as $X_F \in \mathbb{R}^{N \times (N_F + 1)}$, (where' + 1' corresponds to free term in the regression model) and the face shape space $X_Q$. As subject features, reported weight, age, gender, and body measurements extracted from the registered meshes such as body height, arm length, waist circumference are considered. This set is augmented by including their interactions up to $d = 3^{rd}$ degree. Thus, considering in total $N_F$ personal features, the expanded set corresponds to the terms of the polynomial with degree d built from them. This holds for all features except the ones with lower interactions allowed, like gender. In the following, the augmented set of features are denoted by $X_Q \in \mathbb{R}^{N \times (N_G + 1)}$, where the reader can derive the general formula for $N_G$ using basic combinatorial techniques as

$$N_G = \binom{N_F + d}{d} - 1 \tag{8}$$

which, in the case when the (binary) gender feature is included, becomes

$$N_G = \binom{N_F + d}{d} - 1 - (N_F + 1) \tag{9}$$

Equations (8) and (9) are given for completeness but are not needed to understand the rest of the techniques described herein or run algorithms which can simply count the combinations or use simple statistical techniques such as stars and bars combinatorial techniques. To facilitate the notation, the constant term is included in both $X_F$ and $X_G$, but it is not counted in $N_F$ and $N_G$.

Then, multi-linear regression is performed for the body coefficients Y

$$Y = XB + \varepsilon \tag{10}$$

with four settings of the independent variable $X$, with and without interactions and with and without face coefficients:

(a)

$$X = X_F \in \mathbb{R}^{N \times (N_F + 1)} \tag{11}$$

(b)

$$X = X_G \in \mathbb{R}^{N \times (N_G + 1)} \tag{12}$$

(c)

$$X = [X_F, X_Q] \in \mathbb{R}^{N \times (N_F + 1 + N_Q)} \tag{13}$$

(d)

$$X = [X_G, X_Q] \in \mathbb{R}^{N \times (N_G + 1 + N_Q)} \qquad (14)$$

Next, the predictions of specific body parts are evaluated, using the segmentation masks shown in Fig. 3. The arms were excluded from the segmentation masks (including the full body mask) since the test subjects in the population dataset had visible variability in the arm positions and there was no pose model. To improve the prediction for each body part, instead of solving the basic regression Equation (10), the weighted versions as shown below were solved. Let $I_m \in \mathbb{R}^{3N_P \times 3N_P}$ be the diagonal matrix of mask m, where $I_m(j, j)$ = 1 if and only if the vertex is part of the segmentation mask. Recall according to Equation (6) $P = \overline{P}_r + YD'$, and note that for each body part $m$ it is desired to have $I_m P$ accurately predicted. Then, assuming the regression model $Y = XB$, the following is obtained

$$Y\Sigma_m = XB\Sigma_m + \varepsilon\Sigma_m \qquad (15)$$

where $\Sigma_m = D'I_m D \in \mathbb{R}^{200 x 200}$. The least mean square estimate of $B$ in the above equation is

$$\hat{B}_m = ((X'X)^{-1}X'Y\Sigma_m)\Sigma_m^{-1} \qquad (16)$$

for each mask m.

<u>Fitness measures</u> - For each model and mask, a leave-one-out cross validation was performed on the $N$ test subjects. In other words, the estimation of $\hat{B}$ has been carried out every time, leaving out the test subject to predict. Once the predicted body coefficients $\hat{Y}$ are computed, it is necessary to convert back, decode, using the PCA transformation in equation (6) to reach the predicted vertices $\hat{P}$ as

$$\hat{P} = \overline{P}_r + \hat{Y}D' = \overline{P}_r + X\hat{B}D' \qquad (17)$$

To evaluate the prediction, the predicted $\hat{P}(i,:)$ is first aligned to the original coordinates $P(i,:)\forall i \in [1, N]$ with weighted Procrustes (as described in "Principal Warps: Thin-Plate Splines and the Decomposition of Deformations" by F. L. Bookstein, IEEE Transactions on Pattern Analysis and Machine Intelligence, 1989), and then the vertex-wise RMSE is computed over all test subjects for each vertex $v_{i,j}$ versus its predicted position $\hat{v}_{i,j}$

$$E_j = \sqrt{\frac{1}{N}\sum_{i=1}^{N}\left\|\hat{v}_{i,j} - v_{i,j}\right\|_2^2} \qquad (18)$$

As a final measure of fitness for the masks, the mean absolute error E is used for all vertices:

$$E = \frac{1}{N_P}\sum_{j=1}^{N_P}|E_j| \qquad (19)$$

The above error measure also penalizes misplacement of the body part points on the surface and therefore can be considered more accurate.

<u>Results</u> - Two groups of features are evaluated, that are listed in Table 1 below, with twelve features in total.

Table 1

| Name | Type | Mean + Std | Source |
|---|---|---|---|
| Gender | Male[1] or Female[2] | $1.53 \pm 0.5$ | CQ |
| Age | Yrs | $38\text{-}00 \pm 12.59$ | CQ |

(continued)

| Name | Type | Mean + Std | Source |
|---|---|---|---|
| Weight | Kg | 74.56 ± 18.09 | CQ |
| Height | Y-length [mm] | 1701.38 ± 100.78 | PM |
| Waist | Circumference [mm] | 889.83 ± 150.45 | PM |
| Arm | Circumference[mm] | 306.00 ± 43.45 | PM |
| Hip | Circumference[mm] | 1037.99 ± 106.07 | PM |
| Leg | Circumference[mm] | 614.94 ± 67.91 | PM |
| Neck | Circumference[mm] | 364.09 ± 43.33 | PM |
| Leg | Y-distance[mm] | 764.4:1 ± 55.67 | PM |
| Arm | distance [mm] | 557.64 ± 40. 59 | PM |
| Upper Body | Y-distance[mm] | 750.72 ± 42.90 | PM |

The first group is composed of reported gender, age and weight (without clothes), all acquired in the paper "The CAESAR project: a 3-D surface anthropometry survey" mentioned above. The second group includes parametric measurements that were computed from the registered body meshes: the height computed as head to floor; upper body height as head to the highest touchable point of the pelvis; arm length as the distance between acromion (shoulder) to the distal end of the middle finger; leg length from crotch to floor; the perimeters for waist as the midpoint between the lower margin of the last palpable rib and the top of the iliac crest; hips circumference it is performed at the most prominent point, on the major trochanters, and at the level of the maximum relief of the gluteal muscles; arm circumference taken from the midpoint of the total length of the arm, between acromion and olecranon; leg quadriceps circumference taken from the midpoint of the total length of the thigh; neck circumference taken from the midpoint of the total length of the neck.

The features correlation matrix of all the features is presented in Table 2 below.

| | Age | Gender | Weight | Height | WaistC | ArmC | HipC | LegC | NeckC | LegL | ArmL | UBodyH |
|---|---|---|---|---|---|---|---|---|---|---|---|---|
| Age | 1.00 | 0.01 | 0.22 | -0.08 | 0.40 | 0.26 | 0.26 | 0.12 | 0.26 | -0.17 | -0.03 | -0.06 |
| Gender | | 1.00 | -0.42 | -0.64 | -0.34 | -0.31 | 0.03 | 0.01 | -0.68 | -0.39 | -0.65 | -0.57 |
| Weight | | | 1.00 | 0.55 | 0.90 | 0.88 | 0.82 | 0.78 | 0.84 | 0.26 | 0.55 | 0.55 |
| Height | | | | 1.00 | 0.32 | 0.31 | 0.19 | 0.14 | 0.52 | 0.86 | 0.91 | 0.85 |
| WaistC | | | | | 1.00 | 0.85 | 0.82 | 0.74 | 0.83 | 0.02 | 0.36 | 0.36 |
| ArmC | | | | | | 1.00 | 0.82 | 0.81 | 0.76 | 0.01 | 0.31 | 0.36 |
| HipC | | | | | | | 1.00 | 0.92 | 0.56 | -0.03 | 0.19 | 0.24 |
| LegC | | | | | | | | 1.00 | 0.52 | -0.09 | 0.14 | 0.20 |
| NeckC | | | | | | | | | 1.00 | 0.20 | 0.55 | 0.53 |
| LegL | | | | | | | | | | 1.00 | 0.83 | 0.57 |
| ArmL | | | | | | | | | | | 1.00 | 0.68 |
| UBodyH | | | | | | | | | | | | 1.00 |

Table 2

The importance of each feature is assessed by performing a search over all possible combinations of the set $X_F$ resulting in $2^{12}$ = 4096 possible subsets of features. The empty subset is considered as the error compared to the average of the population dataset. For each subset, four different feature designs are compared: $X_F$ (age, gender, height and weight), $X_G$ (which is the set of augmented features determined according to equations (8) and (9) above, e.g. if $X_F$

= {age, weight}, then $X_G$ ={age, age$^2$, age$^3$, age*weight$^2$, age$^2$*weight, weight, weight$^2$, weight$^3$, etc.), [$X_F$,$X_Q$] (age, gender, height and weight, and the facial coefficients $X_Q$) and [$X_G$, $X_Q$] ($X_G$ and the facial coefficients $X_Q$). The maximum number of features reached by models without the face is $N_G$ minus all combinations of the gender. In this example, the maximum number of features is $N_F$=12 and all combinations of gender from second order are $N_F$ + 1. Hence equation (9) provides that the maximum number of regressors, when using interactions, is $N_G$ = 441. Considering instead the example with age, gender, weight and height, where $N_F$ = 4, $N_G$ = 29.

In the following, the mean absolute vertices error $E$ (in mm) for the full body mask (without arms) are presented, along with the errors $E$ for all the remaining four body parts represented by the masks in Fig. 3.

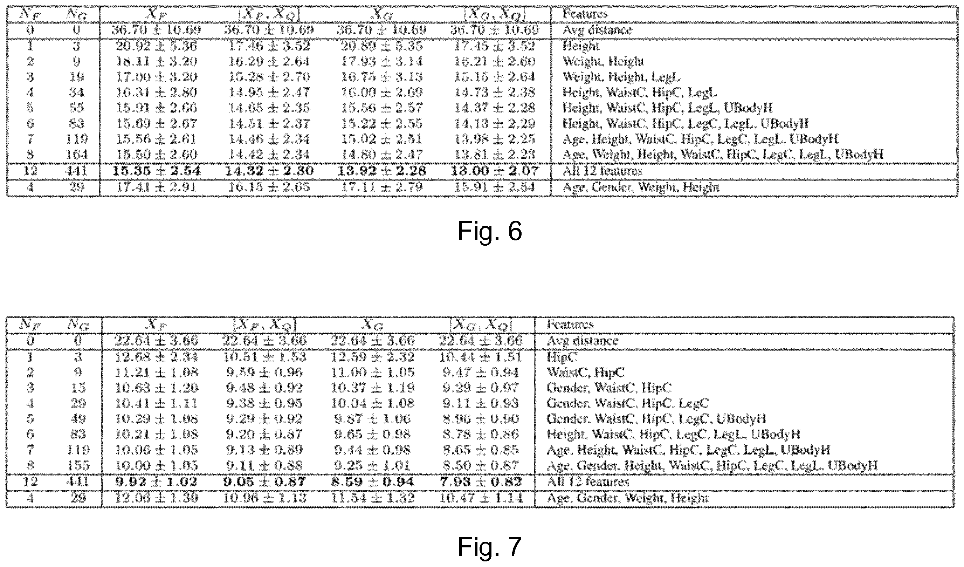

Full body mask without arms - The table in Fig. 6 shows for each cardinality of $X_F$ the best model for $X = X_G$ for the full body mask (without arms) and the error using the other three input matrices. It can be seen that the most accurate single feature is the height with error $E$ = 20.89mm, because it is a major indicator of body size. The only feature that actually outperforms height is the body volume. However, body volume is not easy to measure in a real life scenario, hence the embodiments described herein in which the derived 3D full body representation can be used to estimate the body volume or body part volume. The best combination of two features is the height and the weight, resulting in $E$ = 17.93mm residual error. The minimal error is achieved using all 12 features, and it is 13.92mm for $X_G$ and 13.00mm for [$X_G$, $X_Q$]. The average error reduction for those 12 best models is 1.33mm, and the average effect of adding the face coefficients $X_Q$ is the drop of the error with 8.12%.

In order to evaluate the significance of adding the face shape, consider the model with $X$ = [$X_G$, $X_Q$] where $X_G$ is augmented from = [age, gender, weight, height]. This model has an error of 15.91mm which is better than the error of the model with $N_F$ = 4 best predictors without the face parameters. Therefore this indicates that the face shape can be used instead of detailed parametric measurements. Thus, for example, the face coefficients combined with age, gender and weight features gives a lower error than the prediction using waist, hip circumference and leg length features. It can be seen in Fig. 6 that including features relating to the face has a significant positive contribution to the prediction reliability for all possible subsets of features. In fact, the average error drop, when extending $X_G$ to [$X_G$,$X_Q$], is 0.98mm or 9.72%. On the other hand, the more features that are considered the bigger the effect of adding interactions between them, which can be seen when comparing the $X_F$ column to the $X_G$ column.

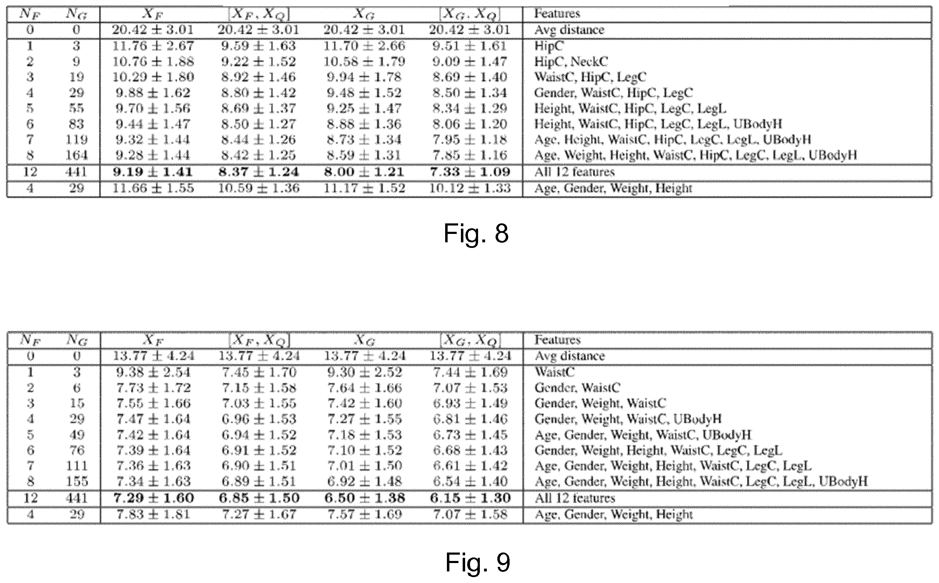

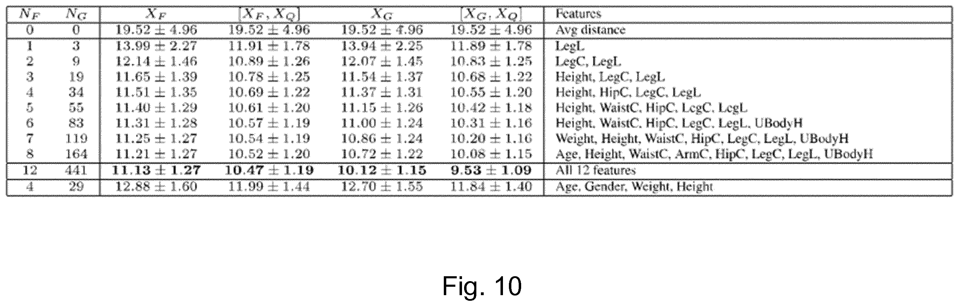

Other body parts - The tables in Figs. 7-10 show for each cardinality of $X_F$ the best model for $X = X_G$ for the other body part masks (Fig. 7 the waist band, Fig. 8 the hips band, Fig. 9 the breasts and Fig. 10 the legs) and the error using the other three input matrices.

It can be seen in Figs. 7 and 8 that while height is the most reliable single feature for the full body (excluding arms), it is not the case for the hips and waist band prediction, where weight gives a better accuracy among the single feature predictors. As would be expected, the circumferences play a much more significant role in the specific masks compared to the full body mask.

For both the waist and hips masks, the best performing feature is the hip circumference, registering an error $E$ of 12.59mm for the waist surface area and 11.70mm for the hip surface area. The lowest error reached using all features for the waist mask is 8.59mm, whereas the hip mask achieved a minimum error of 8.00mm.

For the breast mask, the results for which are shown in Fig. 9, the best single feature is the waist circumference that provides an error of 9.30mm, and as expected, gender plays an important role as well. For this mask the lowest error achieved using all features is 6.50mm.

Finally, from the errors registered for the leg mask (Fig. 10), it can be seen that the leg length is the most useful single parameter, providing an error of 13.94mm. It is followed by the leg circumference and the height. The minimum error achieved in this mask, using all the features, is 10.12mm.

Overall, the use of the face features improves the estimation most for the hips band, where the reduction for the best 12 models is 10.45% (0.99mm). For the waist mask the average reduction is 9.71% (0:98mm) and for the full body the drop is 8.12% (1.33mm). Finally the reduction for the legs is 7.32% (0.84mm) and the face achieves the lowest reduction for the breasts area with 7.14% (0.54mm).

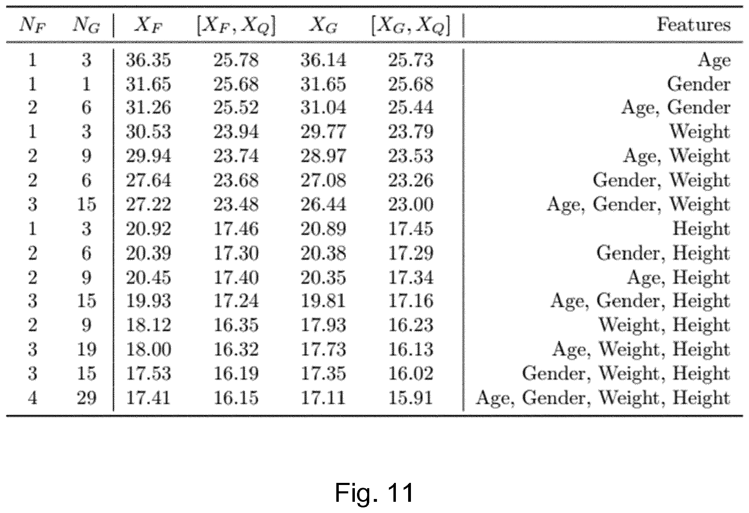

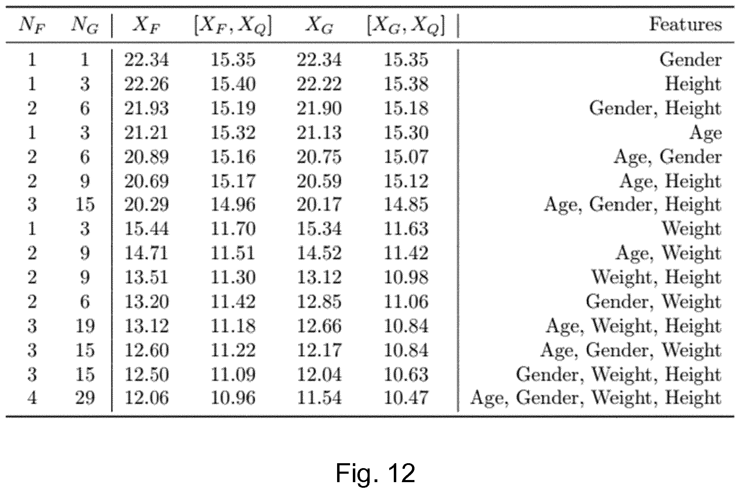

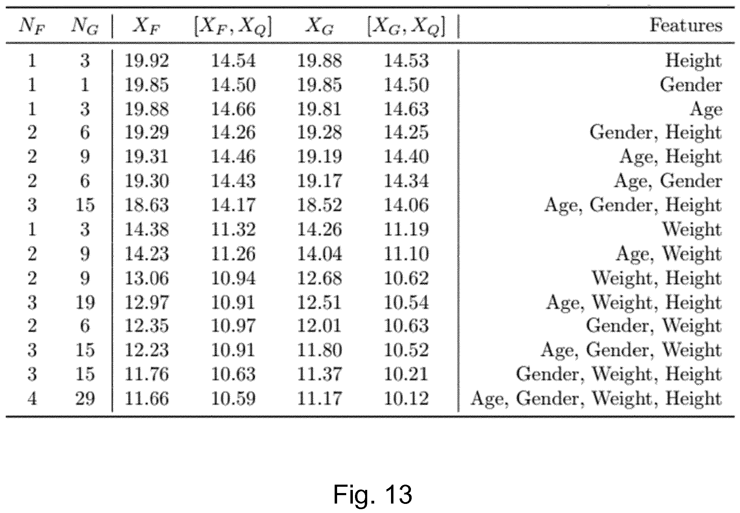

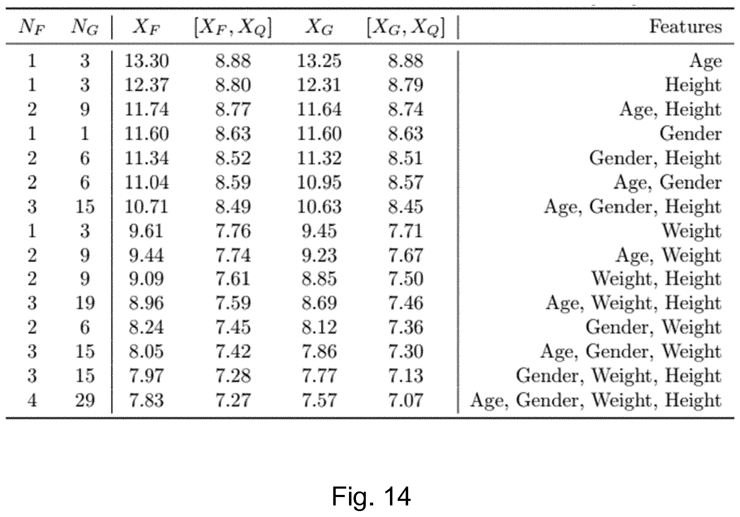

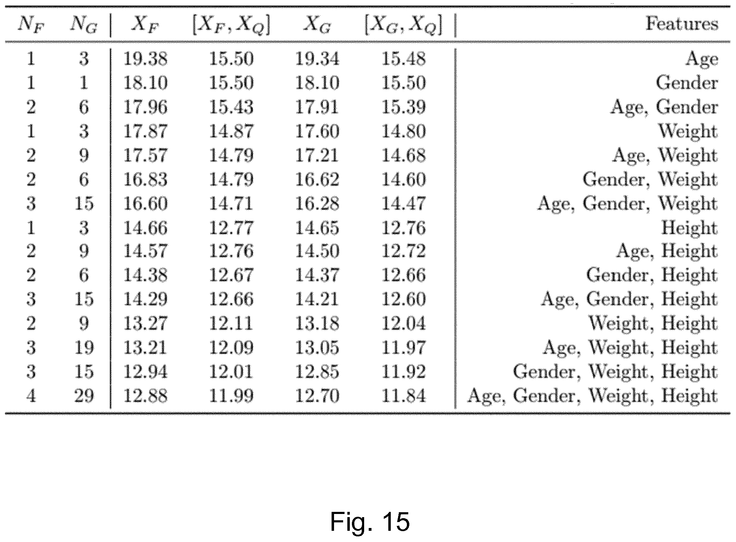

The table in Fig. 11 shows for each cardinality of $X_F$ the best model for $X = X_G$ for the full body mask (without arms) and the error using the other three input matrices for all possible combinations of the subset [age, gender, weight, height] $\subset$ F. The errors $E$ are sorted according to $X_G$ as in Figs. 6-10. The tables in Figs. 12-15 show the corresponding results for the other body part masks (Fig. 12 the waist band, Fig. 13 the hips band, Fig. 14 the breasts and Fig. 15 the legs).

Thus, it can be seen in Figs. 6-15 that the face parameters are a useful addition when determining a body model or body part model, and that the face parameters in combination with other easily measurable or obtainable characteristics (e.g. age, gender, weight and/or height) provides comparable (or sufficient) accuracy to the use of more direct measurements of the body, such as waist/hip circumference, leg length, etc.

Moreover, it can be seen from Figs. 11-15 that while the use of all four of age, gender, weight and height provides the lowest error, the performance of different subsets of these features varies depending on the body mask being considered. For example, weight and height provide the 4th best outcome for the full body mask (without arms) and the legs, but they only provide the 5th best result for the waist band and hips band masks, and the 6th best result

for the breasts mask. Therefore, depending on the part of the body of the subject that is being evaluated, it is possible to use a selected one or more of the characteristics from the set of age, gender, weight and height in combination with the face parameters to achieve an output of a sufficient accuracy. Of course, all of age, gender, weight and height can be used in combination with the face parameters to provide the best accuracy (without extending the modelling to use other specific measurements of the body).

<u>Estimating surface area and/or volume</u> - This part of the description relates to the techniques for estimating the surface area and/or volume of a body part, or of the full body, that make use of the prediction model described above that predicts body shape coefficients based on the face shape space of the subject and one or more of the characteristics age, gender, weight and height.

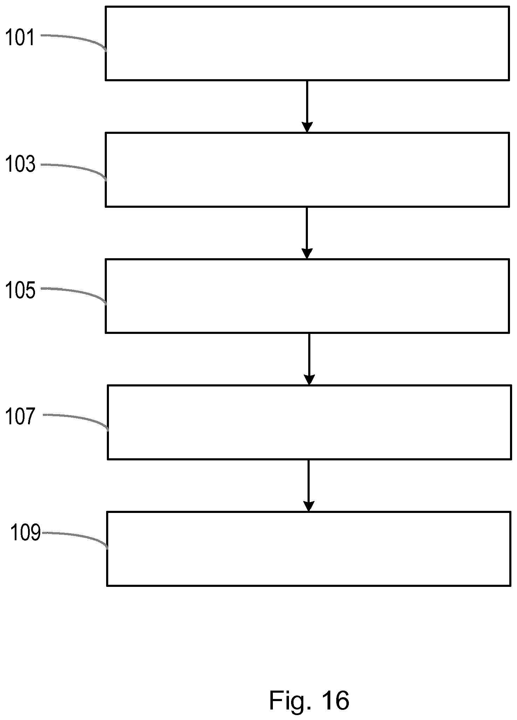

The flow chart in Fig. 16 illustrates an exemplary method for providing a body parametric model, a facial parametric model and a prediction model that can be used for determining a 3D representation of the body or body part(s), from which a surface area and/or volume of a body or a body part of a subject can be estimated. One or more of the steps of the method (although not necessarily all steps) can be performed by the processing unit 8 in the apparatus 4, in conjunction with any of the memory unit 10, interface circuitry 12, user interface 14 and imaging unit 6, as appropriate. The processing unit 8 may perform the one or more steps in response to executing computer program code, that can be stored on a computer readable medium, such as, for example, the memory unit 10.

In a first step, step 101, a body parametric model (e.g. in the form of an avatar) is registered in to a set of 3D body scans (e.g. contained in a population dataset that includes 3D body scans for a number of different test subjects) that includes the body part(s) of interest (e.g. the arms, legs, full body, etc.). Step 101 can be performed, for example, using the techniques described in the paper "The space of human body shapes: reconstruction and parameterization from range scans" mentioned above, or in "Exploring the space of human body shapes: Data-driven synthesis under anthropometric control" by Allen, B., Curless, B., and Popovic, Z., SAE International Proc. Digital Human Modeling for Design and Engineering Conference 2004. The body parametric model relates values of one or more body shape parameters to a 3D representation of the full body (or alternatively to a specific body part or parts). The body parametric model can be a linear model. Step 101 corresponds to the 'Registration' section above.

In step 103, each registered avatar (i.e. the body parametric model adapted to a 3D scan of a particular test subject) is converted to a plurality of body shape parameters (e.g. 200 shape parameters). These body shape parameters can encode the 3D body surface with millimeter accuracy, e.g. corresponding to PCA coefficients, and thus there is a set of values of body shape parameters for each of the test subjects. Step 103 can be performed as described in "The space of human body shapes: reconstruction and parameterization from range scans" and "Exploring the space of human body shapes: Data-driven synthesis under anthropometric control". Step 103 corresponds to the 'Parametric spaces' section above.

In step 105, a facial parametric model (e.g. in the form of an avatar for the face) is registered in to a set of 3D face scans (e.g. that are in a population dataset). Step 105 can be performed, for example using the techniques described in "The space of human body shapes: reconstruction and parameterization from range scans" and "Exploring the space of human body shapes: Data-driven synthesis under anthropometric control". The facial parametric model relates values of one or more facial shape parameters to a 3D representation of the face. The facial parametric model can be a linear model. Step 105 corresponds to the 'Registration' section above.

In step 107, each registered facial avatar (i.e. the facial parametric model adapted to a 3D scan of a particular test subject) is converted to a plurality of facial shape parameters (e.g. 180 facial shape parameters). These facial shape parameters can encode the 3D facial surface with millimeter accuracy, e.g. corresponding to PCA coefficients, and thus there is a set of values of facial shape parameters for each of the test subjects. Step 107 can be performed as described in "The space of human body shapes: reconstruction and parameterization from range scans" and "Exploring the space of human body shapes: Data-driven synthesis under anthropometric control". Step 107 corresponds to the 'Parametric spaces' section above.

In step 109, a prediction model that is to be used to predict a shape representation of each subject (in the form of values for one or more body shape parameters) is built, trained or formed as a function of one or more characteristics and facial shape parameters. The trained or formed prediction model is to receive values of facial shape parameters for a subject and values of the one or more characteristics for the subject as input, and predict values of one or more body shape parameters for the subject (or, where the prediction model includes a body parametric model, predict the 3D representation of the full body or body part by inputting the predicted values of the body shape parameter(s) into the body parametric model). The prediction model is built, trained or formed from the sets of body shape parameters obtained in step 103, the sets of facial shape parameters obtained in step 107, and values of the one or more characteristics for the test subjects in the population dataset.

The prediction model can be a non-linear model. For example the prediction model can be a regression model, such as a cubic polynomial. Step 109 corresponds to the 'Prediction model' section above.

The one or more characteristics that the prediction model is a function of can be any one or more of age of the subject, gender of the subject, weight of the subject and height of the subject. In some embodiments, in addition to the one or more of age, gender, weight and height, the characteristics can include one or more other measurements of the body of the subject, such as leg length, leg (quadriceps) circumference, waist circumference, hips circumference, arm length, arm circumference, upper body height, and neck circumference (all as shown in Fig. 2). The addition of these further characteristics can improve the accuracy of the prediction model in predicting the body shape parameters.

In some embodiments, in step 109 a plurality of candidate prediction models can be formed, with each candidate prediction model using a respective subset of the one or more characteristics. For example a first candidate prediction model can be formed that uses age, gender and height, a second candidate prediction model can be formed that uses gender and weight, a second candidate prediction model can be formed that just uses height, etc. The accuracy (i.e. error) of each of the candidate prediction models in predicting the 3D representation of the full body or a body part or body parts or interest is then evaluated (e.g. with the results shown in Figs. 11-15). In some embodiments, the prediction model formed in step 109 can be the candidate prediction model that provides the highest accuracy (lowest error) of the candidate prediction models. In alternative or further embodiments where it is desired to minimize the number of characteristics required by the prediction model, the prediction model formed in step 109 can be a candidate prediction model that provides a sufficient accuracy with a minimum number of characteristics.

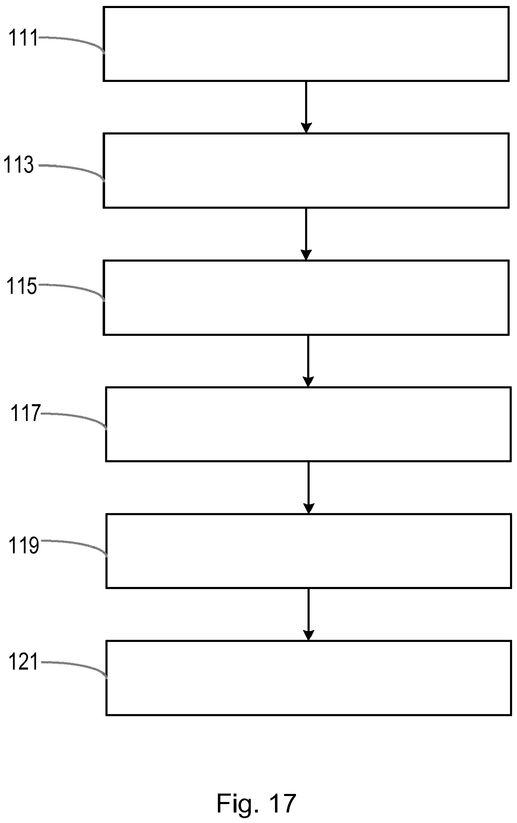

The flow chart in Fig. 17 illustrates an exemplary method for estimating a surface area or volume of a body or a body part of a subject of interest (i.e. a subject not in the population of test subjects). This method makes use of the body parametric model, facial parametric model and prediction model derived in the flow chart of Fig. 16. One or more of the steps of the method in Fig. 17 (although not necessarily all steps) can be performed by the processing unit 8 in the apparatus 4, in conjunction with any of the memory unit 10, interface circuitry 12, user interface 14 and imaging unit 6, as appropriate. The processing unit 8 may perform the one or more steps in response to executing computer program code, that can be stored on a computer readable medium, such as, for example, the memory unit 10.

In a first step, step 111, at least one image of the subject is obtained. The at least one image should include (i.e. show) the face of the subject. In some embodiments a plurality of images can be obtained, or a video sequence of the subject can be obtained (where frames of the video sequence correspond to images). Preferably the at least one image was taken from the front of the subject (i.e. the image(s) show the face 'front-on'). However, it is possible for a plurality of images to be obtained that show the face of the subject from different directions (e.g. an image that shows the left side of the face, an image that shows the right side, etc.). In step 111 the image(s) can be obtained from the imaging unit 6, i.e. the imaging unit 6 is used to capture the image(s) of the face of the subject, and the image(s) are provided to the processing unit 8. Alternatively, in step 111 the processing unit 8 can obtain the image(s) from the memory unit 10, for example if the image(s) were obtained previously by the imaging unit 6 or by another imaging unit/device. In some embodiments, the image(s) can be so-called 'selfies' that are obtained by the subject using the imaging unit 6, their smartphone, or other electronic device with a user-facing imaging unit. The one or more image(s) may be color images or monochrome images.

In step 113, the processing unit 8 processes the at least one image to determine values for one or more facial image parameters for the face of the subject. The values of the one or more facial image parameters represent the 3D geometry of the subject's face.

In this step, an image of the subject's face (which can be a single image or a frame in a video sequence) can be processed to extract a set of two-dimensional (2D) points that represent the facial geometry. The paper "Supervised Descent Method and its Applications to Face Alignment" by Xuehan Xiong Fernando De la Torre, The Robotics Institute, Carnegie Mellon University, Pittsburgh PA, 15213 provides an exemplary technique that can be used to extract 2D points representing facial geometry from an image of a face. These 2D points can then be used to compute a set of 3D points, to provide an avatar representing the face of the subject. The use of the 2D points in this way is described in WO 2017/085075.

Alternatively, in step 113, a video sequence including the subject's face can be processed to extract a set of 3D points that represent the facial geometry. These 3D points provide an avatar representing the face of the subject. This step can use the techniques described in "Dense 3D Face Alignment from 2D Videos in Real-Time" by L'aszl'o A. Jeni (Robotics Institute, Carnegie Mellon University, Pittsburgh, PA, USA), Jeffrey F. Cohn (Robotics Institute and Department of Psychology, University of Pittsburgh, Pittsburgh, PA, USA) and Takeo Kanade (Robotics Institute).

In step 115 (which can be performed before step 111, before 113, or after step 113), values for one or more characteristics of the subject are determined. The one or more characteristics comprises one or more of age, weight, height and gender of the subject. Step 115 can comprise the subject or another person manually entering the value(s) for the one or more characteristics into the apparatus 4, for example using the user interface 14. For example, the subject or another person can manually enter any one or more of the age, weight, height and gender of the subject. Alternatively, step 115 can comprise obtaining or retrieving the value(s) for the one or more characteristics from memory unit 10, for example if the value(s) have previously been stored in the memory unit 10, or from another database, e.g. health records for the subject. Alternatively, step 115 can comprise determining the value(s) for the one or more characteristics by analyzing the image(s) obtained in step 111. For example, the image(s) can be processed to estimate the age of the subject, estimate the weight of the subject (although it will be appreciated that the image(s) should preferably show the subject's face and at least part of their body), estimate the gender of the subject and estimate the height of the subject (although it will be appreciated that the image(s) should show the subject's body). Those skilled in the art of image processing techniques will be aware of suitable techniques that can be used to process image(s) or a video sequence to determine any of age, gender, weight and height. It will be appreciated that in some embodiments where values for a plurality of characteristics are required, a combination of the above embodiments can be used, e.g. a value for one or more of the characteristics (e.g. height) can be input by the subject or another user, a value for another one of the characteristics (e.g. age, or a date of birth) can be stored in the memory unit 10, and/or a value for another one of the characteristics (e.g. gender) can be determined from the image(s) or video sequence.

The specific characteristics for which values are determined in step 115 can depend on the requirements of the prediction model that is to be used in subsequent steps to predict the body shape parameters. For example, in some implementations the prediction model may require values for all four characteristics, whereas in other implementations the prediction model may require values of two specific characteristics, e.g. height and age. As noted further below, in some embodiments the characteristic(s) used by the prediction model can depend on the body part(s) for which the surface area and/or volume is to be estimated.

In step 117, a facial parametric model and the values for the one or more facial image parameters extracted from the image(s) in step 113 are used to determine values for one or more facial shape parameters for the face of the subject. The facial parametric model relates specific values for one or more facial image parameters to a respective 3D representation of a face having respective values for the one or more facial shape parameters. The facial shape parameters can be, for example, PCA coefficients. The facial parametric model can be a linear model, as described above. It should be noted that some types of facial image parameters can be considered as facial shape parameters, and the facial parametric model may not be required to relate all values of facial image parameters to facial shape parameters in step 117.

In particular embodiments of step 117, the facial parametric model is registered in to the image(s) of the face of the subject as represented by the one or more facial image parameters determined in step 113, and the registered facial parametric model is converted into the one or more facial shape parameters.

In step 119, a 3D representation of the full body of the subject is predicted using the prediction model (e.g. as determined in step 109), the values for the one or more characteristics determined in step 105 and the values of the one or more facial shape parameters determined in step 107. The 3D representation of the full body predicted in step 119 indicates the surface shape of the full body.

As noted above, in some embodiments the prediction model uses values of facial shape parameters for a subject and values of the one or more characteristics for the subject as inputs, and predicts values of one or more body shape parameters for the subject. In these embodiments, the values for the one or more body shape parameters are used to predict a 3D representation of the full body using a body parametric model (e.g. as described above with reference to step 101/103) that relates a 3D representation to values of body shape parameters.

In alternative embodiments, the prediction model can also include a body parametric model, in which case the prediction model determines the 3D representation of the full body by inputting the predicted values of the body shape parameter(s) into the body parametric model.

In step 121, the predicted 3D representation of the full body of the subject is analyzed to estimate the surface area and/or volume of the body or body part of the subject (as required). In particular, the surface of the 3D representation will be made up of a (large) plurality of polygons (e.g. triangles obtained by the triangulation of the average vertex positions in the parametric body model), and the surface area of the body or body part can be estimated by summing the areas of the polygons making up that body or body part. Likewise, the 3D representation can be made up of a large plurality of voxels (e.g. tetrahedrons obtained by the tetrahedralisation of the average vertex positions in the parametric body model), and the volume of the body or body part can be estimated by summing the volumes of the voxels making up that body or body part.

Once determined, the estimated surface area and/or volume of the body or body part can be used in one or more of a number of different ways.

In some embodiments, the estimated surface area and/or volume can be output to the subject or another user via the user interface 14. For example the estimated surface area and/or volume can be displayed on a display screen, output as audio via a loudspeaker, etc.

In some embodiments, the estimated surface area and/or volume can be communicated from the apparatus 4 to another electronic device (e.g. via the interface circuitry 12) where it can be stored (e.g. in a health record database) or used by the electronic device.

In some embodiments, the estimated surface area and/or volume can be used or applied in a particular field, such as healthcare (including personal care), online shopping and the textile industry. In the latter two examples, the estimated surface area and/or volume be used to select items of clothing that are a suitable size for the subject.

In the healthcare domain, the estimated surface area can be used to assess the Psoriasis Area and Severity Index (PASI) in a subject with Psoriasis. The estimated surface area and/or volume can also or alternatively be used to determine a drug dosage, such as a chemotherapy dosage.

In the personal care domain, a device can be used to perform a treatment operation on the subject. Many types of device are available that can be used on a body of a subject to provide a treatment operation to the body or a personal care operation on the body. For example, there are devices for the removal of unwanted hairs using various techniques such as shaving, electrolysis, plucking, laser and light therapies (known as photoepilation) and injection of therapeutic anti-androgens. Other types of dermatological treatments, including hair growth reduction and treating acne, can also use light-based technologies. Devices can also be used for providing a massage to the subject, for providing a nail treatment, for providing physiotherapy, for applying patches to the subject (e.g. electrocardiogram electrodes, etc.). Another type of device is an ultrasound probe.

For any of these types of device or treatment operation, it may be useful to know the surface area and/or volume of the body or relevant body part, as this can be used to provide feedback on a treatment operation by a treatment device. Thus, a method of providing feedback is provided in which the surface area and/or volume of the full body or body part of interest is determined using the above method(s), and the estimated surface area and/or volume is used to determine feedback on the treatment operation. For example, knowledge of the surface area of a leg would enable the computation of the average number of light pulses/flashes that is required for epilating the whole leg. This is particularly useful for applications where it is difficult to monitor which areas of the skin have been treated (e.g. in photoepilation the treated areas are invisible to the human eye), and the information can be used to provide the subject or user with (real-time) feedback on the treatment progress. Thus, in some embodiments the processing unit 8 determines a number of treatments (e.g. light pulses/flashes) required to treat a particular body part (or the whole body) based on the estimated surface area and the surface area treated per treatment (e.g. area treated per pulse/flash). The number of treatments may also take into account an amount of overlap between treatments (e.g. the area of one treatment may overlap with a previous treatment area by a certain amount, e.g. 20%). In these embodiments, the processing unit 8 can use the determined number of treatments required for treating the body part (or whole body), along with information on the number of treatments already administered, to indicate to the subject or user how many treatments (e.g. flashes/pulses) are required to complete the treatment operation. The processing unit 8 can present this information to the subject or user in any desired form, e.g. a number of treatments remaining, a percentage completion, etc. This information, including the information about the number of treatments performed and/or the amount of the body part/body treated, can be stored in a treatment record for the subject.

In addition to feedback on the treatment progress, so-called 'smart scheduling' can be used in which the surface area of specific body parts is known, and the subject or other user of the treatment device can be guided through a certain treatment scheme based on factors such as available treatment time (for instance set by the subject or the user) and/or flash device charge.

In some embodiments, the method of estimating the surface area and/or volume in Fig. 17 further comprises a step in which the processing unit 8 receives an indication of what part(s) of the body the surface area and/or volume is to be estimated for. The indication can indicate that the surface area and/or volume is to be estimated for the whole body, the whole body excluding the head, the whole body excluding arms, the legs, a leg, the arms, an arm, a foot, both feet, a hand, both hands, a palm of a hand, both palms, breasts, the waist, the hips, chest, torso, abdomen, back, upper body, lower body, etc. The indication can be an input received from the subject or user, for example via the user interface 14. In some embodiments, the indication can be provided by the subject or user selecting the body or body part from a list of body parts presented or displayed via the user interface 14. In alternative embodiments, the user interface 14 can be used to display the 3D representation of the subject's body determined in step 119 (or alternatively it can display a generic 3D representation of a body), and the subject or user can select one or more parts of the displayed body. The processing unit 8 can then calculate the surface area and/or volume of the part(s) selected by the subject or user. For example, the subject or user could highlight (e.g. by touching on a touchscreen) one or more part(s) of the displayed body, and the processing unit 8 can determine the surface area and/or volume of that/those highlighted parts. In some embodiments, the user interface 14 may enable the subject or user to highlight only predetermined body parts (or the full body), for example similar to the templates shown in Fig. 3. However, in other embodiments, the user interface 14 may enable the subject or user to select or highlight any desired part or combination of parts of the body. For example the user or subject could highlight only a portion of a body part shown in the templates of Fig. 3, or multiple portions or parts of the body (e.g. the user or subject could select the lower arms, or the left arm and left leg, etc.). In this respect, the subject or user may be able to select multiple body parts that are non-contiguous (i.e. not adjacent or touching each other). The subject or user may highlight or select parts of the body that are relevant to a particular treatment operation.

As noted above with respect to the performance measures shown in Figs. 11-15, while the use of all four of age, gender, weight and height (in combination with the facial shape parameters) provides the lowest error, the performance of different subsets of these features varies depending on the body mask being considered. Therefore, in some embodiments, based on the body part (including the full body) for which the surface area and/or volume is to be estimated, the one or more characteristics that are determined or required in step 115 can be adapted. More specifically, the prediction model to use to predict the 3D representation can be adapted (or an appropriate candidate prediction model determined in embodiments of step 109 selected) based on the body part(s) to be evaluated. In this way, the amount of information required to be derived or input by the subject or other user can be reduced, while still providing an acceptable accuracy for the surface area and/or volume estimation. Alternatively, the minimum amount of information that is to be derived or input by the subject or other user can be determined that provides a certain level of accuracy for the surface area and/or volume estimation.

For example, if it is desired to only use two characteristics in step 115 (and subsequent steps), then if the surface area and/or volume of the legs or full body (excluding arms) is to be estimated, then step 115 may comprise determining the weight and height, as they provide the most accurate two-characteristic result for these two masks. Whereas, if the surface area and/or volume of the hips, waist or breasts is to be estimated, then step 115 may comprise determining the gender and weight instead. As another example, it may be determined that a particular combination of characteristics is sub-optimum when compared to another combination of characteristic(s), in which case it may be better to use that/those other combination of characteristic(s) instead. E.g. for the hips band weight provides a more accurate 3D representation than age, gender and height combined. In that case, it is preferred to use weight as the characteristic for that prediction model rather than the other three characteristics, or to at least make sure that weight is included in the combination of characteristics.

Therefore, depending on the part of the body of the subject that is to be evaluated, it is possible to use a selected one or more of the characteristics from the set of age, gender, weight and height in combination with the face shape parameters to achieve an output of a sufficient accuracy, or to minimize the amount of information that needs to be manually input, retrieved from another information source and/or determined from the image(s).

As noted above, in some embodiments the user or subject can dynamically select the part or parts of the body for which the surface area and/or volume is to be estimated, e.g. by highlighting or selecting one or more portions or parts of a body on a user interface 14. In this case an 'optimum' set of input characteristics to use for estimating the 3D representation of that/those body part(s) may not be known. Therefore, in these embodiments, before proceeding with the method in Fig. 17, the method can perform step 109 to determine a prediction model that is able to predict the 3D representation of the highlighted or selected body part(s). The prediction model will be a function of a specific one or more of the characteristics that are determined to be the most appropriate or most reliable for the selected body part(s) and the facial shape parameters. Once the prediction model is determined, the values of the characteristic(s) required for that prediction model can be received from the user or subject (in some embodiments this/these value(s) can be received following a request to the user or subject to provide the value(s) of those characteristics), or otherwise determined in step 115. Thus, in these embodiments the algorithm is able to determine new prediction models as required based on a selection of a body part or body parts by the user or subject.

In some embodiments, the accuracy of the facial shape parameter(s) determined in step 117 can be improved by also using the values of the one or more characteristics determined in step 115 to determine the values of the one or more facial shape parameters. In these embodiments, a facial prediction model can be determined in step 107 of Fig. 16 that receives facial image parameters (e.g. that can be obtained from an image of the face of the subject) and the one or more characteristics (i.e. age, gender, weight and/or height) as input and outputs predicted values of the facial shape parameters.

Therefore there is provided improved techniques for estimating the surface area and/or volume of a body part or body of a subject without requiring a 3D body scan or 3D body part scan of the subject. Instead, the disclosed techniques require an image of the face of the subject and limited (but easily obtainable) information on the subject, such as age, gender, weight and/or height.

Variations to the disclosed embodiments can be understood and effected by those skilled in the art in practicing the principles and techniques described herein, from a study of the drawings, the disclosure and the appended claims. In the claims, the word "comprising" does not exclude other elements or steps, and the indefinite article "a" or "an" does not exclude a plurality. A single processor or other unit may fulfil the functions of several items recited in the claims. The mere fact that certain measures are recited in mutually different dependent claims does not indicate that a combination of these measures cannot be used to advantage. A computer program may be stored or distributed on a suitable medium, such as an optical storage medium or a solid-state medium supplied together with or as part of other hardware, but may also be distributed in other forms, such as via the Internet or other wired or wireless telecommunication systems. Any reference signs in the claims should not be construed as limiting the scope.

**Claims**

1. A computer-implemented method for estimating a surface area and/or a volume of a body or a body part of a subject, the method comprising:

   obtaining at least one image, wherein the at least one image includes a face of the subject;

processing the at least one image to determine values for one or more facial image parameters for the face of the subject;

determining values for one or more characteristics of the subject, wherein the one or more characteristics comprises one or more of age of the subject, weight of the subject, height of the subject and gender of the subject; using a facial parametric model and the determined values for the one or more facial image parameters to determine values for one or more facial shape parameters for the face of the subject, wherein the facial parametric model relates specific values for one or more facial image parameters to a respective 3D representation of a face having respective values for the one or more facial shape parameters;

using a prediction model with the determined values for the one or more characteristics and the determined values of the one or more facial shape parameters to predict a 3D representation of the full body of the subject; and analyzing the predicted 3D representation of the full body of the subject to estimate the surface area and/or the volume of the body or body part of the subject.

2. A method as claimed in claim 1, wherein the step of using the prediction model to predict the 3D representation of the full body comprises:

using the prediction model to predict values of one or more body shape parameters from the determined values for the one or more characteristics and the determined values of the one or more facial shape parameters; and using a body parametric model and the predicted values of the one or more body shape parameters to predict the 3D representation of the full body, wherein the body parametric model relates specific values for the one or more body shape parameters to a respective 3D representation of a body.

3. A method as claimed in claim 1 or 2, wherein the method further comprises: determining the prediction model from a population dataset, wherein the population dataset comprises 3D scans of a plurality of test subjects and values for the one or more characteristics for each of the test subjects, and the prediction model is determined by:

registering a body parametric model to each of the 3D scans, wherein the body parametric model relates a 3D representation of a body to specific values of one or more body shape parameters; determining values of the one or more body shape parameters for each of the registered body parametric models; registering a facial parametric model to each of the 3D scans, wherein the facial parametric model relates a 3D representation of a face to specific values of one or more facial shape parameters; determining values of the one or more facial shape parameters for each of the registered facial parametric models; and

forming the prediction model from the determined values of the one or more body shape parameters, determined values of the one or more facial shape parameters and values for the one or more characteristics for each of the test subjects.

4. A method as claimed in any of claims 1-3, wherein the prediction model is specific to the body part of the subject for which the surface area and/or the volume is to be estimated, wherein the prediction model predicts the 3D representation of the full body based on determined values of the one or more facial shape parameters and a respective subset of the one or more characteristics.

5. A method as claimed in claim 3 or 4, wherein the step of forming the prediction model comprises:

forming a plurality of candidate prediction models, wherein each candidate prediction model uses a respective subset of the one or more characteristics; evaluating an accuracy of each of the candidate prediction models in predicting the 3D representation of the full body or a body part or body parts; and forming the prediction model as a candidate prediction model that provides one of: a highest accuracy of the candidate prediction models, and/or a sufficient accuracy with a minimum number of characteristics.

6. A method as claimed in any of claims 1-5, wherein the method further comprises:

receiving an indication of the body or the body part of the subject for which the surface area and/or the volume is to be estimated; wherein the step of analyzing comprises analyzing the predicted 3D representation of the full body of the subject to estimate the surface area and/or the volume of the indicated body or body part of the subject.

**7.** A method as claimed in claim 6, wherein the method further comprises:
requesting an input indicating the body or body part of the subject for which the surface area and/or the volume is to be estimated.

**8.** A method as claimed in claim 6 or 7 when dependent on claim 4 or 5, wherein the method further comprises:
requesting an input indicating the values of the respective subset of the one or more characteristics used by the prediction model for the indicated body part.

**9.** A method as claimed in any of claims 6-8 when dependent on claim 4 or 5, wherein the step of forming the prediction model is performed after receiving the indication.

**10.** A method as claimed in any of claims 1-9, wherein the step of determining values for the plurality of characteristics comprises processing the at least one image to determine the values for one or more of the plurality of characteristics.

**11.** A method as claimed in any of claims 1-9, wherein the step of determining values for the one or more characteristics comprises receiving an input from the subject indicating the values for one or more of the one or more characteristics.

**12.** A computer-implemented method of providing feedback on a treatment operation by a treatment device, the method comprising:

estimating the surface area and/or volume of a body or a body part of a subject that is to be treated in a treatment operation using the treatment device according to the method of any of claims 1-11; and
using the estimated surface area and/or volume to determine feedback on the treatment operation.

**13.** A computer program product comprising a computer readable medium having computer readable code embodied therein, the computer readable code being configured such that, on execution by a suitable computer or processor, the computer or processor is caused to perform the method of any of claims 1-12.

**14.** An apparatus for estimating a surface area and/or a volume of a body or a body part of a subject, the apparatus comprising a processing unit is configured to:

obtain at least one image from an imaging unit, wherein the at least one image includes a face of the subject;
process the at least one image to determine values for one or more facial image parameters for the face of the subject;
determine values for one or more characteristics of the subject, wherein the one or more characteristics comprises one or more of age of the subject, weight of the subject, height of the subject and gender of the subject;
use a facial parametric model and the determined values for the one or more facial image parameters to determine values for one or more facial shape parameters for the face of the subject, wherein the facial parametric model relates specific values for one or more facial image parameters to a respective 3D representation of a face having respective values for the one or more facial shape parameters;
use a prediction model with the determined values for the one or more characteristics and the determined values of the one or more facial shape parameters to predict a 3D representation of the full body of the subject; and
analyze the predicted 3D representation of the full body of the subject to estimate the surface area and/or the volume of the body or body part of the subject.

**15.** A system, comprising:

an apparatus as claimed in claim 14; and
an imaging unit for obtaining the at least one image.

Fig. 1

Fig. 2

Fig. 3

Fig. 4

(a)

(b)

Fig. 5

| $N_F$ | $N_G$ | $X_F$ | $[X_F, X_Q]$ | $X_G$ | $[X_G, X_Q]$ | Features |
|---|---|---|---|---|---|---|
| 0 | 0 | $36.70 \pm 10.69$ | $36.70 \pm 10.69$ | $36.70 \pm 10.69$ | $36.70 \pm 10.69$ | Avg distance |
| 1 | 3 | $20.92 \pm 5.36$ | $17.46 \pm 3.52$ | $20.89 \pm 5.35$ | $17.45 \pm 3.52$ | Height |
| 2 | 9 | $18.11 \pm 3.20$ | $16.29 \pm 2.64$ | $17.93 \pm 3.14$ | $16.21 \pm 2.60$ | Weight, Height |
| 3 | 19 | $17.00 \pm 3.20$ | $15.28 \pm 2.70$ | $16.75 \pm 3.13$ | $15.15 \pm 2.64$ | Weight, Height, LegL |
| 4 | 34 | $16.31 \pm 2.80$ | $14.95 \pm 2.47$ | $16.00 \pm 2.69$ | $14.73 \pm 2.38$ | Height, WaistC, HipC, LegL |
| 5 | 55 | $15.91 \pm 2.66$ | $14.65 \pm 2.35$ | $15.56 \pm 2.57$ | $14.37 \pm 2.28$ | Height, WaistC, HipC, LegL, UBodyH |
| 6 | 83 | $15.69 \pm 2.67$ | $14.51 \pm 2.37$ | $15.22 \pm 2.55$ | $14.13 \pm 2.29$ | Height, WaistC, HipC, LegC, LegL, UBodyH |
| 7 | 119 | $15.56 \pm 2.61$ | $14.46 \pm 2.34$ | $15.02 \pm 2.51$ | $13.98 \pm 2.25$ | Age, Height, WaistC, HipC, LegC, LegL, UBodyH |
| 8 | 164 | $15.50 \pm 2.60$ | $14.42 \pm 2.34$ | $14.80 \pm 2.47$ | $13.81 \pm 2.23$ | Age, Weight, Height, WaistC, HipC, LegC, LegL, UBodyH |
| 12 | 441 | $\mathbf{15.35 \pm 2.54}$ | $\mathbf{14.32 \pm 2.30}$ | $\mathbf{13.92 \pm 2.28}$ | $\mathbf{13.00 \pm 2.07}$ | All 12 features |
| 4 | 29 | $17.41 \pm 2.91$ | $16.15 \pm 2.65$ | $17.11 \pm 2.79$ | $15.91 \pm 2.54$ | Age, Gender, Weight, Height |

Fig. 6

| $N_F$ | $N_G$ | $X_F$ | $[X_F, X_Q]$ | $X_G$ | $[X_G, X_Q]$ | Features |
|---|---|---|---|---|---|---|
| 0 | 0 | $22.64 \pm 3.66$ | $22.64 \pm 3.66$ | $22.64 \pm 3.66$ | $22.64 \pm 3.66$ | Avg distance |
| 1 | 3 | $12.68 \pm 2.34$ | $10.51 \pm 1.53$ | $12.59 \pm 2.32$ | $10.44 \pm 1.51$ | HipC |
| 2 | 9 | $11.21 \pm 1.08$ | $9.59 \pm 0.96$ | $11.00 \pm 1.05$ | $9.47 \pm 0.94$ | WaistC, HipC |
| 3 | 15 | $10.63 \pm 1.20$ | $9.48 \pm 0.92$ | $10.37 \pm 1.19$ | $9.29 \pm 0.97$ | Gender, WaistC, HipC |
| 4 | 29 | $10.41 \pm 1.11$ | $9.38 \pm 0.95$ | $10.04 \pm 1.08$ | $9.11 \pm 0.93$ | Gender, WaistC, HipC, LegC |
| 5 | 49 | $10.29 \pm 1.08$ | $9.29 \pm 0.92$ | $9.87 \pm 1.06$ | $8.96 \pm 0.90$ | Gender, WaistC, HipC, LegC, UBodyH |
| 6 | 83 | $10.21 \pm 1.08$ | $9.20 \pm 0.87$ | $9.65 \pm 0.98$ | $8.78 \pm 0.86$ | Height, WaistC, HipC, LegC, LegL, UBodyH |
| 7 | 119 | $10.06 \pm 1.05$ | $9.13 \pm 0.89$ | $9.44 \pm 0.98$ | $8.65 \pm 0.85$ | Age, Height, WaistC, HipC, LegC, LegL, UBodyH |
| 8 | 155 | $10.00 \pm 1.05$ | $9.11 \pm 0.88$ | $9.25 \pm 1.01$ | $8.50 \pm 0.87$ | Age, Gender, Height, WaistC, HipC, LegC, LegL, UBodyH |
| 12 | 441 | $\mathbf{9.92 \pm 1.02}$ | $\mathbf{9.05 \pm 0.87}$ | $\mathbf{8.59 \pm 0.94}$ | $\mathbf{7.93 \pm 0.82}$ | All 12 features |
| 4 | 29 | $12.06 \pm 1.30$ | $10.96 \pm 1.13$ | $11.54 \pm 1.32$ | $10.47 \pm 1.14$ | Age, Gender, Weight, Height |

Fig. 7

| $N_F$ | $N_G$ | $X_F$ | $[X_F, X_Q]$ | $X_G$ | $[X_G, X_Q]$ | Features |
|---|---|---|---|---|---|---|
| 0 | 0 | $20.42 \pm 3.01$ | $20.42 \pm 3.01$ | $20.42 \pm 3.01$ | $20.42 \pm 3.01$ | Avg distance |
| 1 | 3 | $11.76 \pm 2.67$ | $9.59 \pm 1.63$ | $11.70 \pm 2.66$ | $9.51 \pm 1.61$ | HipC |
| 2 | 9 | $10.76 \pm 1.88$ | $9.22 \pm 1.52$ | $10.58 \pm 1.79$ | $9.09 \pm 1.47$ | HipC, NeckC |
| 3 | 19 | $10.29 \pm 1.80$ | $8.92 \pm 1.46$ | $9.94 \pm 1.78$ | $8.69 \pm 1.40$ | WaistC, HipC, LegC |
| 4 | 29 | $9.88 \pm 1.62$ | $8.80 \pm 1.42$ | $9.48 \pm 1.52$ | $8.50 \pm 1.34$ | Gender, WaistC, HipC, LegC |
| 5 | 55 | $9.70 \pm 1.56$ | $8.69 \pm 1.37$ | $9.25 \pm 1.47$ | $8.34 \pm 1.29$ | Height, WaistC, HipC, LegC, LegL |
| 6 | 83 | $9.44 \pm 1.47$ | $8.50 \pm 1.27$ | $8.88 \pm 1.36$ | $8.06 \pm 1.20$ | Height, WaistC, HipC, LegC, LegL, UBodyH |
| 7 | 119 | $9.32 \pm 1.44$ | $8.44 \pm 1.26$ | $8.73 \pm 1.34$ | $7.95 \pm 1.18$ | Age, Height, WaistC, HipC, LegC, LegL, UBodyH |
| 8 | 164 | $9.28 \pm 1.44$ | $8.42 \pm 1.25$ | $8.59 \pm 1.31$ | $7.85 \pm 1.16$ | Age, Weight, Height, WaistC, HipC, LegC, LegL, UBodyH |
| 12 | 441 | $\mathbf{9.19 \pm 1.41}$ | $\mathbf{8.37 \pm 1.24}$ | $\mathbf{8.00 \pm 1.21}$ | $\mathbf{7.33 \pm 1.09}$ | All 12 features |
| 4 | 29 | $11.66 \pm 1.55$ | $10.59 \pm 1.36$ | $11.17 \pm 1.52$ | $10.12 \pm 1.33$ | Age, Gender, Weight, Height |

Fig. 8

| $N_F$ | $N_G$ | $X_F$ | $[X_F, X_Q]$ | $X_G$ | $[X_G, X_Q]$ | Features |
|---|---|---|---|---|---|---|
| 0 | 0 | $13.77 \pm 4.24$ | $13.77 \pm 4.24$ | $13.77 \pm 4.24$ | $13.77 \pm 4.24$ | Avg distance |
| 1 | 3 | $9.38 \pm 2.54$ | $7.45 \pm 1.70$ | $9.30 \pm 2.52$ | $7.44 \pm 1.69$ | WaistC |
| 2 | 6 | $7.73 \pm 1.72$ | $7.15 \pm 1.58$ | $7.64 \pm 1.66$ | $7.07 \pm 1.53$ | Gender, WaistC |
| 3 | 15 | $7.55 \pm 1.66$ | $7.03 \pm 1.55$ | $7.42 \pm 1.60$ | $6.93 \pm 1.49$ | Gender, Weight, WaistC |
| 4 | 29 | $7.47 \pm 1.64$ | $6.96 \pm 1.53$ | $7.27 \pm 1.55$ | $6.81 \pm 1.46$ | Gender, Weight, WaistC, UBodyH |
| 5 | 49 | $7.42 \pm 1.64$ | $6.94 \pm 1.52$ | $7.18 \pm 1.53$ | $6.73 \pm 1.45$ | Age, Gender, Weight, WaistC, UBodyH |
| 6 | 76 | $7.39 \pm 1.64$ | $6.91 \pm 1.52$ | $7.10 \pm 1.52$ | $6.68 \pm 1.43$ | Gender, Weight, Height, WaistC, LegC, LegL |
| 7 | 111 | $7.36 \pm 1.63$ | $6.90 \pm 1.51$ | $7.01 \pm 1.50$ | $6.61 \pm 1.42$ | Age, Gender, Weight, Height, WaistC, LegC, LegL |
| 8 | 155 | $7.34 \pm 1.63$ | $6.89 \pm 1.51$ | $6.92 \pm 1.48$ | $6.54 \pm 1.40$ | Age, Gender, Weight, Height, WaistC, LegC, LegL, UBodyH |
| 12 | 441 | $\mathbf{7.29 \pm 1.60}$ | $\mathbf{6.85 \pm 1.50}$ | $\mathbf{6.50 \pm 1.38}$ | $\mathbf{6.15 \pm 1.30}$ | All 12 features |
| 4 | 29 | $7.83 \pm 1.81$ | $7.27 \pm 1.67$ | $7.57 \pm 1.69$ | $7.07 \pm 1.58$ | Age, Gender, Weight, Height |

Fig. 9

| $N_F$ | $N_G$ | $X_F$ | $[X_F, X_Q]$ | $X_G$ | $[X_G, X_Q]$ | Features |
|---|---|---|---|---|---|---|
| 0 | 0 | $19.52 \pm 4.96$ | $19.52 \pm 4.96$ | $19.52 \pm 4.96$ | $19.52 \pm 4.96$ | Avg distance |
| 1 | 3 | $13.99 \pm 2.27$ | $11.91 \pm 1.78$ | $13.94 \pm 2.25$ | $11.89 \pm 1.78$ | LegL |
| 2 | 9 | $12.14 \pm 1.46$ | $10.89 \pm 1.26$ | $12.07 \pm 1.45$ | $10.83 \pm 1.25$ | LegC, LegL |
| 3 | 19 | $11.65 \pm 1.39$ | $10.78 \pm 1.25$ | $11.54 \pm 1.37$ | $10.68 \pm 1.22$ | Height, LegC, LegL |
| 4 | 34 | $11.51 \pm 1.35$ | $10.69 \pm 1.22$ | $11.37 \pm 1.31$ | $10.55 \pm 1.20$ | Height, HipC, LegC, LegL |
| 5 | 55 | $11.40 \pm 1.29$ | $10.61 \pm 1.20$ | $11.15 \pm 1.26$ | $10.42 \pm 1.18$ | Height, WaistC, HipC, LegC, LegL |
| 6 | 83 | $11.31 \pm 1.28$ | $10.57 \pm 1.19$ | $11.00 \pm 1.24$ | $10.31 \pm 1.16$ | Height, WaistC, HipC, LegC, LegL, UBodyH |
| 7 | 119 | $11.25 \pm 1.27$ | $10.54 \pm 1.19$ | $10.86 \pm 1.24$ | $10.20 \pm 1.16$ | Weight, Height, WaistC, HipC, LegC, LegL, UBodyH |
| 8 | 164 | $11.21 \pm 1.27$ | $10.52 \pm 1.20$ | $10.72 \pm 1.22$ | $10.08 \pm 1.15$ | Age, Height, WaistC, ArmC, HipC, LegC, LegL, UBodyH |
| 12 | 441 | $\mathbf{11.13 \pm 1.27}$ | $\mathbf{10.47 \pm 1.19}$ | $\mathbf{10.12 \pm 1.15}$ | $\mathbf{9.53 \pm 1.09}$ | All 12 features |
| 4 | 29 | $12.88 \pm 1.60$ | $11.99 \pm 1.44$ | $12.70 \pm 1.55$ | $11.84 \pm 1.40$ | Age, Gender, Weight, Height |

Fig. 10

| $N_F$ | $N_G$ | $X_F$ | $[X_F, X_Q]$ | $X_G$ | $[X_G, X_Q]$ | Features |
|---|---|---|---|---|---|---|
| 1 | 3 | 36.35 | 25.78 | 36.14 | 25.73 | Age |
| 1 | 1 | 31.65 | 25.68 | 31.65 | 25.68 | Gender |
| 2 | 6 | 31.26 | 25.52 | 31.04 | 25.44 | Age, Gender |
| 1 | 3 | 30.53 | 23.94 | 29.77 | 23.79 | Weight |
| 2 | 9 | 29.94 | 23.74 | 28.97 | 23.53 | Age, Weight |
| 2 | 6 | 27.64 | 23.68 | 27.08 | 23.26 | Gender, Weight |
| 3 | 15 | 27.22 | 23.48 | 26.44 | 23.00 | Age, Gender, Weight |
| 1 | 3 | 20.92 | 17.46 | 20.89 | 17.45 | Height |
| 2 | 6 | 20.39 | 17.30 | 20.38 | 17.29 | Gender, Height |
| 2 | 9 | 20.45 | 17.40 | 20.35 | 17.34 | Age, Height |
| 3 | 15 | 19.93 | 17.24 | 19.81 | 17.16 | Age, Gender, Height |
| 2 | 9 | 18.12 | 16.35 | 17.93 | 16.23 | Weight, Height |
| 3 | 19 | 18.00 | 16.32 | 17.73 | 16.13 | Age, Weight, Height |
| 3 | 15 | 17.53 | 16.19 | 17.35 | 16.02 | Gender, Weight, Height |
| 4 | 29 | 17.41 | 16.15 | 17.11 | 15.91 | Age, Gender, Weight, Height |

Fig. 11

EP 3 742 397 A1

| $N_F$ | $N_G$ | $X_F$ | $[X_F, X_Q]$ | $X_G$ | $[X_G, X_Q]$ | Features |
|---|---|---|---|---|---|---|
| 1 | 1 | 22.34 | 15.35 | 22.34 | 15.35 | Gender |
| 1 | 3 | 22.26 | 15.40 | 22.22 | 15.38 | Height |
| 2 | 6 | 21.93 | 15.19 | 21.90 | 15.18 | Gender, Height |
| 1 | 3 | 21.21 | 15.32 | 21.13 | 15.30 | Age |
| 2 | 6 | 20.89 | 15.16 | 20.75 | 15.07 | Age, Gender |
| 2 | 9 | 20.69 | 15.17 | 20.59 | 15.12 | Age, Height |
| 3 | 15 | 20.29 | 14.96 | 20.17 | 14.85 | Age, Gender, Height |
| 1 | 3 | 15.44 | 11.70 | 15.34 | 11.63 | Weight |
| 2 | 9 | 14.71 | 11.51 | 14.52 | 11.42 | Age, Weight |
| 2 | 9 | 13.51 | 11.30 | 13.12 | 10.98 | Weight, Height |
| 2 | 6 | 13.20 | 11.42 | 12.85 | 11.06 | Gender, Weight |
| 3 | 19 | 13.12 | 11.18 | 12.66 | 10.84 | Age, Weight, Height |
| 3 | 15 | 12.60 | 11.22 | 12.17 | 10.84 | Age, Gender, Weight |
| 3 | 15 | 12.50 | 11.09 | 12.04 | 10.63 | Gender, Weight, Height |
| 4 | 29 | 12.06 | 10.96 | 11.54 | 10.47 | Age, Gender, Weight, Height |

Fig. 12

| $N_F$ | $N_G$ | $X_F$ | $[X_F, X_Q]$ | $X_G$ | $[X_G, X_Q]$ | Features |
|---|---|---|---|---|---|---|
| 1 | 3 | 19.92 | 14.54 | 19.88 | 14.53 | Height |
| 1 | 1 | 19.85 | 14.50 | 19.85 | 14.50 | Gender |
| 1 | 3 | 19.88 | 14.66 | 19.81 | 14.63 | Age |
| 2 | 6 | 19.29 | 14.26 | 19.28 | 14.25 | Gender, Height |
| 2 | 9 | 19.31 | 14.46 | 19.19 | 14.40 | Age, Height |
| 2 | 6 | 19.30 | 14.43 | 19.17 | 14.34 | Age, Gender |
| 3 | 15 | 18.63 | 14.17 | 18.52 | 14.06 | Age, Gender, Height |
| 1 | 3 | 14.38 | 11.32 | 14.26 | 11.19 | Weight |
| 2 | 9 | 14.23 | 11.26 | 14.04 | 11.10 | Age, Weight |
| 2 | 9 | 13.06 | 10.94 | 12.68 | 10.62 | Weight, Height |
| 3 | 19 | 12.97 | 10.91 | 12.51 | 10.54 | Age, Weight, Height |
| 2 | 6 | 12.35 | 10.97 | 12.01 | 10.63 | Gender, Weight |
| 3 | 15 | 12.23 | 10.91 | 11.80 | 10.52 | Age, Gender, Weight |
| 3 | 15 | 11.76 | 10.63 | 11.37 | 10.21 | Gender, Weight, Height |
| 4 | 29 | 11.66 | 10.59 | 11.17 | 10.12 | Age, Gender, Weight, Height |

Fig. 13

| $N_F$ | $N_G$ | $X_F$ | $[X_F, X_Q]$ | $X_G$ | $[X_G, X_Q]$ | Features |
|---|---|---|---|---|---|---|
| 1 | 3 | 13.30 | 8.88 | 13.25 | 8.88 | Age |
| 1 | 3 | 12.37 | 8.80 | 12.31 | 8.79 | Height |
| 2 | 9 | 11.74 | 8.77 | 11.64 | 8.74 | Age, Height |
| 1 | 1 | 11.60 | 8.63 | 11.60 | 8.63 | Gender |
| 2 | 6 | 11.34 | 8.52 | 11.32 | 8.51 | Gender, Height |
| 2 | 6 | 11.04 | 8.59 | 10.95 | 8.57 | Age, Gender |
| 3 | 15 | 10.71 | 8.49 | 10.63 | 8.45 | Age, Gender, Height |
| 1 | 3 | 9.61 | 7.76 | 9.45 | 7.71 | Weight |
| 2 | 9 | 9.44 | 7.74 | 9.23 | 7.67 | Age, Weight |
| 2 | 9 | 9.09 | 7.61 | 8.85 | 7.50 | Weight, Height |
| 3 | 19 | 8.96 | 7.59 | 8.69 | 7.46 | Age, Weight, Height |
| 2 | 6 | 8.24 | 7.45 | 8.12 | 7.36 | Gender, Weight |
| 3 | 15 | 8.05 | 7.42 | 7.86 | 7.30 | Age, Gender, Weight |
| 3 | 15 | 7.97 | 7.28 | 7.77 | 7.13 | Gender, Weight, Height |
| 4 | 29 | 7.83 | 7.27 | 7.57 | 7.07 | Age, Gender, Weight, Height |

Fig. 14

| $N_F$ | $N_G$ | $X_F$ | $[X_F, X_Q]$ | $X_G$ | $[X_G, X_Q]$ | Features |
|---|---|---|---|---|---|---|
| 1 | 3 | 19.38 | 15.50 | 19.34 | 15.48 | Age |
| 1 | 1 | 18.10 | 15.50 | 18.10 | 15.50 | Gender |
| 2 | 6 | 17.96 | 15.43 | 17.91 | 15.39 | Age, Gender |
| 1 | 3 | 17.87 | 14.87 | 17.60 | 14.80 | Weight |
| 2 | 9 | 17.57 | 14.79 | 17.21 | 14.68 | Age, Weight |
| 2 | 6 | 16.83 | 14.79 | 16.62 | 14.60 | Gender, Weight |
| 3 | 15 | 16.60 | 14.71 | 16.28 | 14.47 | Age, Gender, Weight |
| 1 | 3 | 14.66 | 12.77 | 14.65 | 12.76 | Height |
| 2 | 9 | 14.57 | 12.76 | 14.50 | 12.72 | Age, Height |
| 2 | 6 | 14.38 | 12.67 | 14.37 | 12.66 | Gender, Height |
| 3 | 15 | 14.29 | 12.66 | 14.21 | 12.60 | Age, Gender, Height |
| 2 | 9 | 13.27 | 12.11 | 13.18 | 12.04 | Weight, Height |
| 3 | 19 | 13.21 | 12.09 | 13.05 | 11.97 | Age, Weight, Height |
| 3 | 15 | 12.94 | 12.01 | 12.85 | 11.92 | Gender, Weight, Height |
| 4 | 29 | 12.88 | 11.99 | 12.70 | 11.84 | Age, Gender, Weight, Height |

Fig. 15

101

103

105

107

109

Fig. 16

Fig. 17

Europäisches
Patentamt

European
Patent Office

Office européen
des brevets

# EUROPEAN SEARCH REPORT

**Application Number**

EP 19 17 6080

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 2017/029488 A2 (METAIL LTD [GB]) 23 February 2017 (2017-02-23) * Section 2. Personalised Head Modelling section 2.1.1 2D Face Localisation and Automatic Landmark Detection section 2.3.2 Head Size Estimation from Body Shape Parameters section 4.3 Requesting Additional Measurements Using a Measurement Selection Process section 4.6 Exploiting the Correlation between User's Face Shape and User's Body Shape; figures 1,2,7,0,33 * | 1-15 | INV. G06T7/60 G06T7/62 |
| A | ENES KOCABEY ET AL: "Face-to-BMI: Using Computer Vision to Infer Body Mass Index on Social Media", ARXIV.ORG, CORNELL UNIVERSITY LIBRARY, 201 OLIN LIBRARY CORNELL UNIVERSITY ITHACA, NY 14853, 9 March 2017 (2017-03-09), XP080755430, * the whole document * | 1-15 | |
| A | US 2018/289334 A1 (DE BROUWER WALTER [US] ET AL) 11 October 2018 (2018-10-11) * the whole document * | 1-15 | |
| A | WO 2015/050929 A1 (PHILADELPHIA CHILDREN HOSPITAL [US]) 9 April 2015 (2015-04-09) * the whole document * | 1-15 | |

TECHNICAL FIELDS
SEARCHED      (IPC)

G06T

The present search report has been drawn up for all claims

| Place of search | Date of completion of the search | Examiner |
|---|---|---|
| Munich | 26 November 2019 | Tillier, Christophe |

CATEGORY OF CITED DOCUMENTS

X : particularly relevant if taken alone
Y : particularly relevant if combined with another
    document of the same category
A : technological background
O : non-written disclosure
P : intermediate document

T : theory or principle underlying the invention
E : earlier patent document, but published on, or
    after the filing date
D : document cited in the application
L : document cited for other reasons

.....................................................................................

& : member of the same patent family, corresponding
    document

EPO FORM 1503 03.82 (P04C01)

## ANNEX TO THE EUROPEAN SEARCH REPORT
## ON EUROPEAN PATENT APPLICATION NO.

EP 19 17 6080

This annex lists the patent family members relating to the patent documents cited in the above-mentioned European search report.
The members are as contained in the European Patent Office EDP file on
The European Patent Office is in no way liable for these particulars which are merely given for the purpose of information.

26-11-2019

| Patent document cited in search report | | Publication date | Patent family member(s) | | | Publication date |
|---|---|---|---|---|---|---|
| WO 2017029488 | A2 | 23-02-2017 | CN | 107924579 | A | 17-04-2018 |
| | | | EP | 3335195 | A2 | 20-06-2018 |
| | | | GB | 2543893 | A | 03-05-2017 |
| | | | US | 2019035149 | A1 | 31-01-2019 |
| | | | WO | 2017029488 | A2 | 23-02-2017 |
| US 2018289334 | A1 | 11-10-2018 | NONE | | | |
| WO 2015050929 | A1 | 09-04-2015 | US | 2016253798 | A1 | 01-09-2016 |
| | | | WO | 2015050929 | A1 | 09-04-2015 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2017085075 A

**Non-patent literature cited in the description**

- **S. WUHRER ; C. SHU.** Estimating 3D human shapes from measurements. *Mach. Vis. Appl.,* 2013, vol. 24 (6), 1133-1147
- **LOPER, M. ; MAHMOOD, N. ; ROMERO, J. ; PONS-MOLL, G. ; BLACK, M. J.** SMPL: A skinned multi-person linear model. *CM transactions on graphics (TOG),* vol. 34 (6), 248
- **B. ALLEN ; B. CURLESS ; Z. POPOVIC.** The space of human body shapes: reconstruction and parameterization from range scans. *ACM Transactions on Graphics,* 2003
- **V. BLANZ ; T. VETTER.** A morphable model for the synthesis of 3D faces. *Proceedings of the 26th annual conference on Computer graphics and interactive techniques,* 1999
- **R. BALL ; J. MOLENBROEK.** Measuring Chinese heads and faces. *Proceedings of the 9th International Congress of Physiological Anthropology, Human diversity: design for life,* 2008
- **K. ROBINETTE ; H. DAANEN ; E. PAQUET.** The CAESAR project: a 3-D surface anthropometry survey. *Second International Conference on 3-D Digital Imaging and Modeling (Cat. No.PR00062),* 1999

- **G. K. TAM ; Z. Q. CHENG ; Y. K. LAI ; F. C. LANGBEIN ; Y. LIU ; D. MARSHALL ; R. R. MARTIN ; X. F. SUN ; P. L. ROSIN.** Registration of 3d point clouds and meshes: A survey from rigid to Nonrigid. *IEEE Transactions on Visualization and Computer Graphics,* 2013
- **O. VAN KAICK ; H. ZHANG ; G. HAMARNEH ; D. COHEN-OR.** A survey on shape correspondence. *Eurographics Symposium on Geometry Processing,* 2011
- **X. LI ; S. S. IYENGAR.** On Computing Mapping of 3D Objects. *ACM Computing Surveys,* 2014
- **F. L. BOOKSTEIN.** Principal Warps: Thin-Plate Splines and the Decomposition of Deformations. *IEEE Transactions on Pattern Analysis and Machine Intelligence,* 1989
- **ALLEN, B. ; CURLESS, B. ; POPOVIC, Z.** Exploring the space of human body shapes: Data-driven synthesis under anthropometric control. *SAE International Proc. Digital Human Modeling for Design and Engineering Conference,* 2004
- **XUEHAN XIONG FERNANDO DE LA TORRE.** Supervised Descent Method and its Applications to Face Alignment. The Robotics Institute, Carnegie Mellon University
- **L'ASZL'O A. JENI.** Dense 3D Face Alignment from 2D Videos in Real-Time. Robotics Institute, Carnegie Mellon University